# EUROPEAN PATENT APPLICATION

(11) **EP 4 415 100 A1**
(43) Date of publication of application: **14.08.2024**
(21) Application number: 22878904.6
(22) Date of filing: 06.10.2022
(51) Int. Cl.: H01M 10/0567, H01M 10/052

(54) **ELECTROLYTE AND SECONDARY BATTERY COMPRISING SAME**

(30) Priority: 06.10.2021 KR 20210132198; 04.10.2022 KR 20220126138
(71) Applicant: Soulbrain Co., Ltd., Seongnam-si, Gyeonggi-do 13486 (KR)
(72) Inventor: KIM, Min Goo, Seongnam-si, Gyeonggi-do 13486 (KR); JANG, Min Jung, Seongnam-si, Gyeonggi-do 13486 (KR); KIM, Jae Yoon, Seongnam-si, Gyeonggi-do 13486 (KR); LEE, Sang Ho, Seongnam-si, Gyeonggi-do 13486 (KR); YUN, Jong Cheol, Seongnam-si, Gyeonggi-do 13486 (KR); HAN, Ji Seong, Seongnam-si, Gyeonggi-do 13486 (KR)
(74) Representative: Dehns
(86) International application number: PCT/KR2022/015027
(87) International publication number: WO 2023/059083

(57) **Abstract**

Disclosed is an electrolyte and a secondary battery containing the same. According to the present disclosure, charging efficiency and output may be improved due to low discharge resistance, and gas generation and thickness increase may be suppressed to provide a secondary battery with long-term lifespan and excellent high temperature capacity retention.

## Description

### TECHNICAL FIELD

The present application claims priority to Korean Patent Application No. 10-2021-0132198 filed on October 6, 2021 in the Republic of Korea and Korean Patent Application No. 10-2022-0126138 filed on October 4, 2022 in the Republic of Korea, the disclosures of which are incorporated herein by reference.

The present disclosure relates to an electrolyte and a secondary battery including the same, and more specifically, to an electrolyte for a battery, which includes an electrolyte additive capable of improving output characteristics and high temperature storage characteristics of the battery and significantly reducing gas generation and thickness increase rate, and a secondary battery including the same.

### BACKGROUND ART

Lithium secondary batteries enable smooth movement of lithium ions by inserting an electrolyte between the positive and negative electrodes, and electricity is generated or consumed through oxidation-reduction reactions due to intercalation and de-intercalation at the positive and negative electrodes.

Lithium secondary batteries, which are mainly used as a power source for mobile IT devices such as mobile phones and power tools, are being used for vehicles and energy storage as large-capacity technology develops.

With the expansion of such application fields and the increase in demand, better battery performance and stability than those required for existing small batteries are required. Also, in recent years, in order to improve battery characteristics such as output characteristics, cycle characteristics, preservation characteristics, and film characteristics, various studies are being conducted on organic solvents and additives as electrolyte components.

In the past, in the case of electrolytes that do not contain an electrolyte additive or contains an electrolyte additive with poor properties, it is difficult to expect improvement in low-temperature output characteristics due to the formation of a non-uniform SEI film. Moreover, even when electrolyte additives are included, if the input amount cannot be adjusted to the required amount, the electrolyte additives may cause decomposition of the positive electrode surface during high temperature reaction or cause the electrolytes to undergo an oxidation reaction, ultimately deteriorating the cycle characteristics and storage stability of the secondary battery.

### RELATED LITERATURES

### Patent Literature

(Patent Literature 1) Korean Patent Registration No. 1295395

### DISCLOSURE

### Technical Problem

The present disclosure is designed to solve the problems of the related art, and therefore the present disclosure is directed to providing an electrolyte for a battery, which contains an electrolyte additive that may improve the output characteristics and high temperature storage characteristics of the battery and significantly reduce gas generation and thickness increase rate.

The present disclosure is also directed to providing an excellent secondary battery that may improve battery output by reducing discharge resistance, improve recovery capacity at high temperature to enable long-term storage, and suppress gas generation in the battery.

These and other purposes of the present disclosure may all be achieved by the following present disclosure.

### Technical Solution

In one aspect of the present disclosure, there is provided an electrolyte, which includes an organic solvent, lithium salt, a first additive and a second additive,
wherein the first additive contains a compound having an atomic group with 3 to 5 atoms represented by Formula 1 or 2 in an amount of 15 weight% or less based on 100 weight% of the electrolyte, the atomic group having 2 to 4 atoms with electronegativity of 3 or more and having at least one double bond,
wherein the second additive contains a compound having an atomic group with 3 to 5 atoms and a symmetric structure in an amount of 0.01 to 10 weight% based on 100 weight% of the electrolyte, the atomic group having 2 to 4 atoms with electronegativity of 3 or more and having at least one double bond. 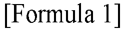 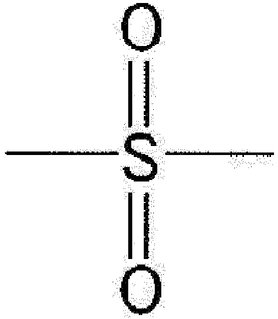 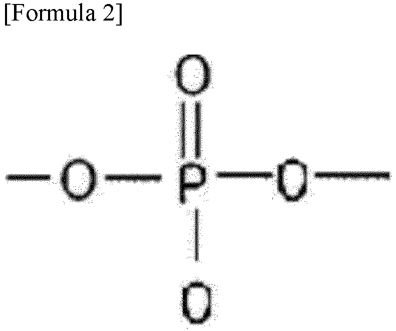
(In Formulas 1 and 2, a solid line is a bond.)

The first additive may be at least one selected from compounds represented by Formulas 3 to 6 below. 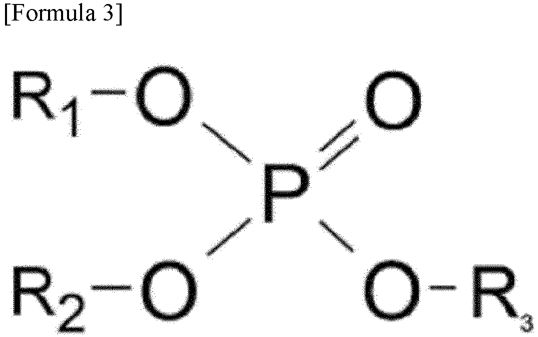 (In Formula 3, R₁, R₂ and R₃ are independently an alkyl group with 1 to 5 carbon atoms or an aryl group with 6 to 10 carbon atoms.) 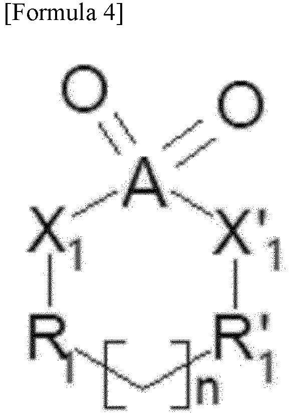 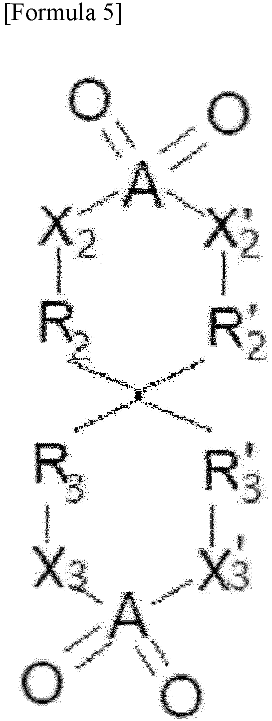 (In Formulas 4 to 5, A is phosphorus (P), sulfur (S), or nitrogen (N); X₁, X₂, X₃, X₁', X₂' and X₃' are independently a bond or oxygen; n is an integer of 0 to 3; and R₁, R₂, R₃, R₁', R₂' and R₃' are independently a bond, substituted or unsubstituted alkylene with 1 to 10 carbon atoms and have at least one carbon.) 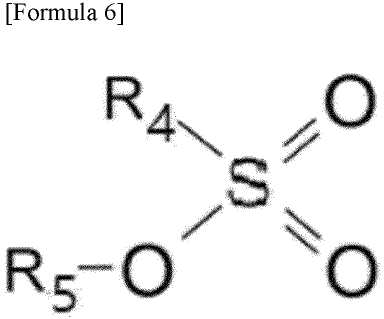 (In Formula 6, R₄ is OR₆ or R₆; R₅ is R₇A; A is 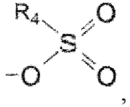 and R₆ and R₇ are independently an alkyl group with 1 to 5 carbon atoms or an aryl group with 6 to 10 carbon atoms.)

The first additive may be at least one selected from compounds represented by Formulas 7 to 13 below. 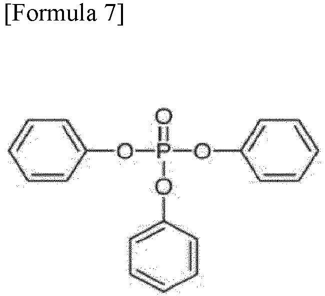 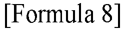 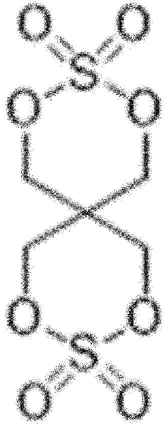 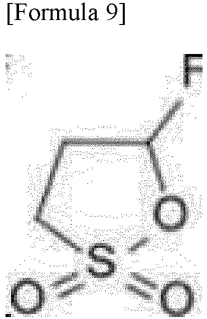 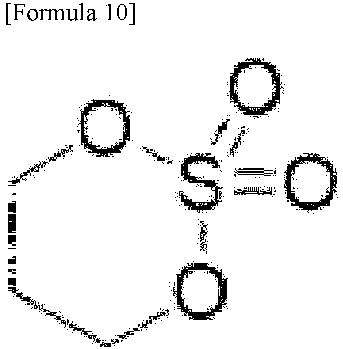 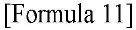 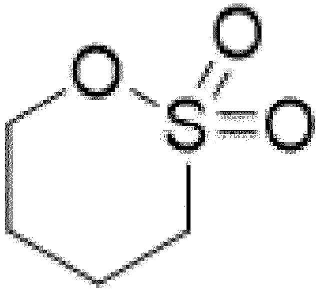 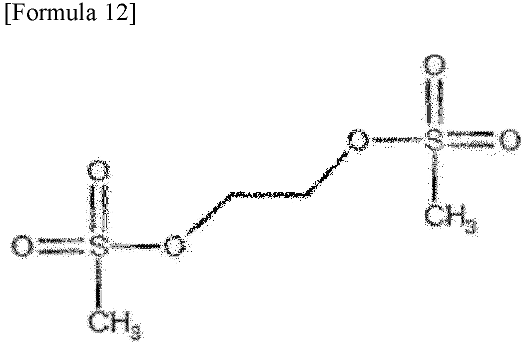 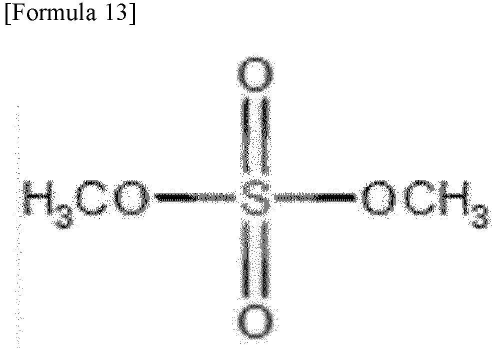 (In Formulas 7 to 13, a solid line is a bond; a point where a bond meets a bond is carbon when a separate element is not described; and the number of hydrogens satisfying the atom value of the carbon is omitted.)

The second additive may be a compound containing a pair of at least one cation selected from the group consisting of alkali metal ions (excluding lithium) and an anion represented by Formula 14 below. 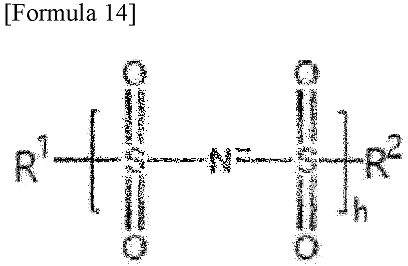 (In Formula 14, R¹ and R² are independently hydrogen, halogen, or a straight-chain or branched alkyl group containing a halogen substituent with 1 to 7 carbon atoms, and h is an integer of 1 to 10.)

In the anion represented by Formula 14, the halogen substituent may be fluorine.

The second additive may be a compound containing a pair of a cation of cesium or sodium and at least one anion selected from the group represented by Formulas 15 to 17 below. 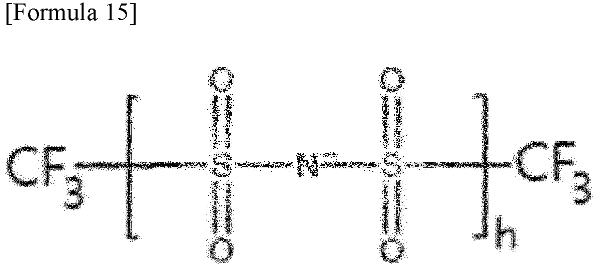 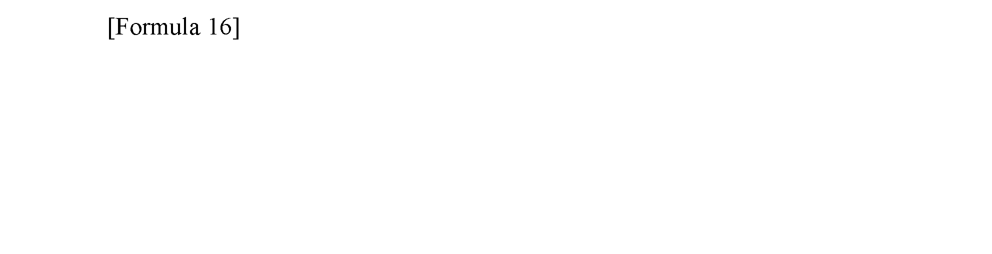 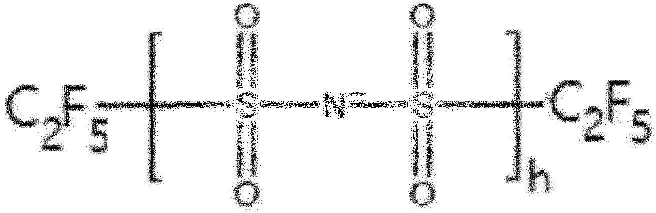 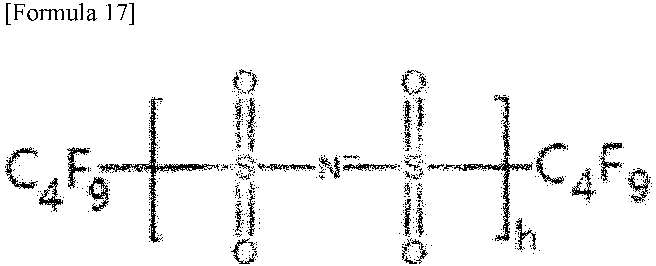 (In Formulas 15 to 17, h is an integer of 1 to 10.)

The first additive and the second additive may be included at a weight ratio of 1:0.5 to 2.5 (first additive: second additive).

The lithium salt may include at least one selected from the group consisting of LiPF₆, LiClO₄, LiAsF₆, LiBF₄, LiBF₆, LiSbF₆, LiAl0₄, LiAlCl₄, LiClO₄, LiCF₃SO₃, LiC₄F₉SO₃, LiN(C₂F₅SO₃)₂, LiN(C₂F₅SO₂)₂, LiN(CF₃SO₂)₂, LiN(CaF₂ₐ₊₁SO₂) (C_{b}F_{2b+1}SO₂) (where a and b are natural numbers), LiCl, LiI, and LiB(C₂O₄)₂.

The organic solvent may include at least two selected from the group consisting of ethylene carbonate (EC), diethyl carbonate (DEC), ethylmethyl carbonate (EMC), dimethyl carbonate (DMC), propylene carbonate (PC), dipropyl carbonate (DPC), butylene carbonate, methylpropyl carbonate, ethylpropyl carbonate, methyl propionate (MP), ethyl propionate (EP), and propyl propionate (PP).

The electrolyte may further comprise at least one third additive selected from the group consisting of boron compound, phosphorus compound, sulfur compound, and nitrogen compound in an amount of 10 weight% or less based on a total of 100 weight% of the electrolyte.

In another aspect of the present disclosure, there is also provided an electrolyte additive, comprising: at least one compound selected from compounds represented by Formulas 3 to 6 below; and a compound containing a pair of cesium cation and an anion represented by Formula 14 below. 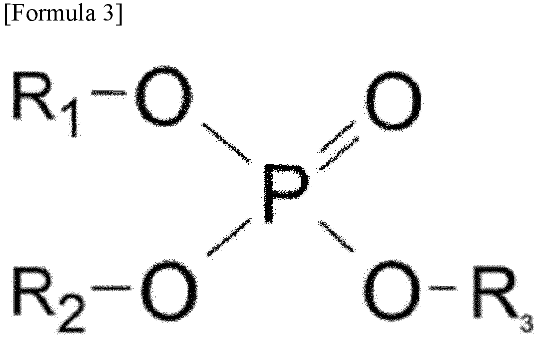 (In Formula 3, R₁, R₂ and R₃ are independently an alkyl group with 1 to 5 carbon atoms or an aryl group with 6 to 10 carbon atoms.) 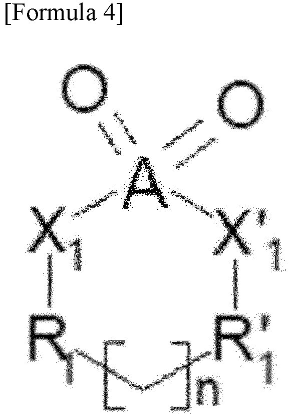 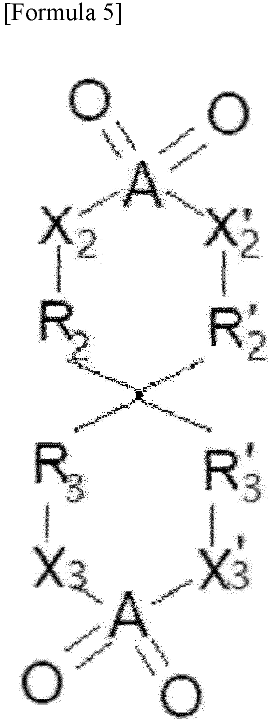 (In Formulas 4 to 5, A is phosphorus (P), sulfur (S), or nitrogen (N); X₁, X₂, X₃, X₁', X₂' and X₃' are independently a bond or oxygen; n is an integer of 0 to 3; and R₁, R₂, R₃, R₁', R₂' and R₃' are independently a bond, substituted or unsubstituted alkylene with 1 to 10 carbon atoms and have at least one carbon.) 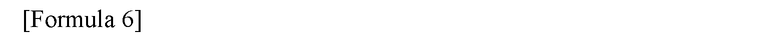 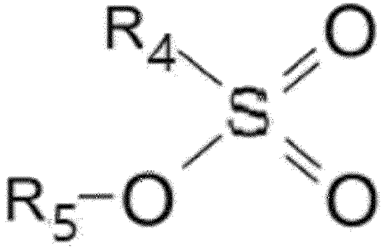 (In Formula 6, R₄ is OR₆ or R₆; R₅ is R₇A; A is 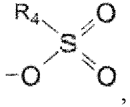 and R₆ and R₇ are independently an alkyl group with 1 to 5 carbon atoms or an aryl group with 6 to 10 carbon atoms.) 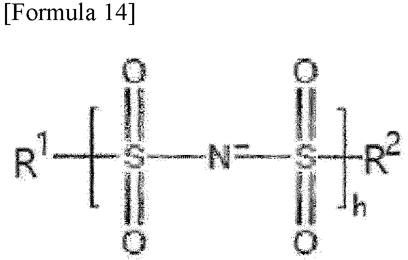 (In Formula 14, R¹ and R² are independently hydrogen, halogen, or a straight-chain or branched alkyl group containing a halogen substituent with 1 to 7 carbon atoms, and h is an integer of 1 to 10.)

The at least one compound selected from compounds represented by Formulas 3 to 6 and the compound containing a pair of cesium cation and an anion represented by Formula 14 may be included at a weight ratio of 1:0.5 to 2.5 (first additive: second additive).

In another aspect of the present disclosure, there is also provided a lithium secondary battery, which includes a negative electrode, a positive electrode, a separator interposed between the negative electrode and the positive electrode, and an electrolyte, wherein the electrolyte may be an electrolyte described above.

The secondary battery may have a discharge resistance value of 28 mΩ or less at 60°C.

The secondary battery may have a recovery capacity of 585 mAh or more at 60°C.

The secondary battery may have a thickness increase rate of 2.6% or less at 60°C calculated using Equation 1 below. Thickness increase rate (%) = {(thickness after high temperature storage - initial thickness)/initial thickness)/initial thickness} X 100

The secondary battery may have a coulombic efficiency of 99.5% or more calculated using Equation 2 below after 300 cycles.Coulombic efficiency (%) = (discharge capacity at 300 cycles / charge capacity at 300 cycles) X 100

In the secondary battery, the cell thickness may increase by less than 4 mm due to the generation of CO₂ gas and H₂ gas due to electrolyte decomposition and SEI film formation during 300 cycle charge and discharge.

The secondary battery may be a battery for an energy storage system (ESS) or a vehicle.

### Advantageous Effects

When an electrolyte for a battery according to the present disclosure is included in a secondary battery, the output may be improved by reducing discharge resistance, and the recovery capacity at high temperature may be improved, thereby providing a secondary battery with excellent long-term lifespan and high temperature capacity retention.

In particular, the additive or the like for a battery according to the present disclosure suppresses gas generation and thickness increase within the battery, so it is possible to provide a secondary battery with excellent performance and lifespan.

### BEST MODE

Hereinafter, an electrolyte additive, an electrolyte for a battery, and a secondary battery containing the same according to the present disclosure will be described in detail.

The inventors of this application have researched secondary batteries with improved output and excellent high temperature recovery capacity and lifespan characteristics in order to manufacture batteries that can be used as vehicle batteries, and they have found that all of the above objectives could be achieved when an additive of a specific structure is added to the electrolyte of the secondary battery. Based on this, the inventors have devoted themselves further to research and completed the present disclosure.

The electrolyte additive of the present disclosure is a compound that includes a first additive and a second additive, wherein the first additive contains a compound having an atomic group with 3 to 5 atoms represented by Formula 1 or 2 in an amount of 15 weight% or less based on 100 weight% of the electrolyte, the atomic group having 2 to 4 atoms with electronegativity of 3 or more and having at least one double bond, and the second additive is a compound having an atomic group represented by Formula 1 below with 3 to 5 atoms and a symmetric structure, the atomic group having 2 to 4 atoms with electronegativity of 3 or more and having at least one double bond. In this case, the charging resistance is reduced, the output of the battery is improved, the recovery capacity at high temperature is improved to enable long-term storage, and an excellent lifespan maintenance rate at high temperature is secured. 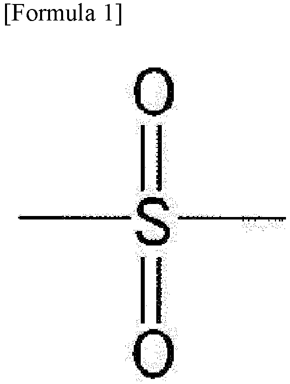 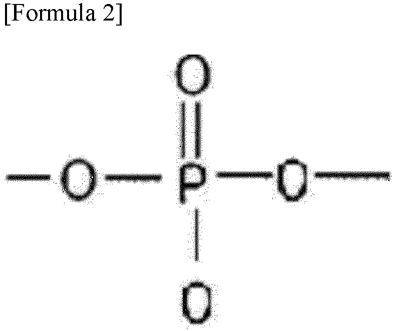 (In Formulas 1 and 2, a solid line is a bond.)

In addition, the electrolyte for a battery according to the present disclosure includes the electrolyte additive.

If the electrolyte additive having an atomic group represented by Formula 1 or 2 is added to the electrolyte of a secondary battery, electrons are localized toward the O element due to the difference in electronegativity between the O elements, which are directly connected to the P or S element. Accordingly, the P or S element becomes electron-poor (e- poor, δ+), which induces an oxidation reaction in the electrolyte containing lithium ions, forming a stable film on the electrode, specifically the positive electrode (cathode). At this time, the decomposition of the electrolyte may be prevented due to the stability of the film, and this may improve cycle characteristics. In particular, since the electrolyte is not decomposed at high temperature, compared to the conventional electrode film that decomposes at high temperature to deteriorate high temperature storage, there is an excellent effect of greatly improving high temperature storage. In addition, an increase in resistance is prevented, which has the effect of improving charging efficiency and output, and gas generation due to chemical reactions inside the battery is also suppressed, thereby improving the safety of the battery. In addition, capacity retention is improved by preventing collapse of the electrode active material structure of the positive electrode and the negative electrode at high temperature, which has the effect of extending lifespan.

The first additive may be at least one compound selected from compounds represented by Formulas 3 to 6 below. If a lithium secondary battery is constructed using an electrolyte containing the first additive, the terminal group of the corresponding compound has the effect of suppressing the decomposition of the electrolyte, thereby reducing the rate of increase in internal resistance and reducing the amount of gas generated and the rate of increase in thickness, which has the effect of extending the lifespan of the battery. 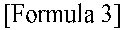 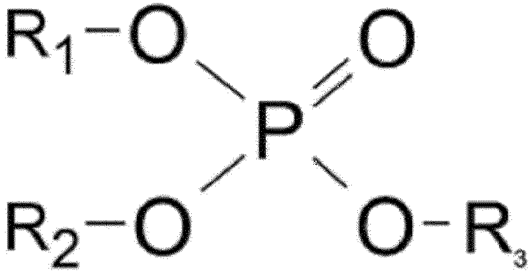 (In Formula 3, R₁, R₂ and R₃ are independently an alkyl group with 1 to 5 carbon atoms or an aryl group with 6 to 10 carbon atoms.) 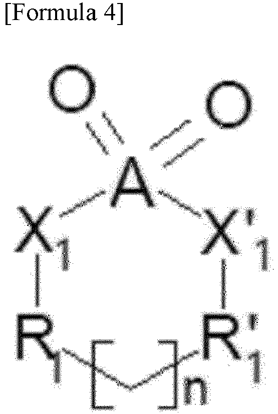 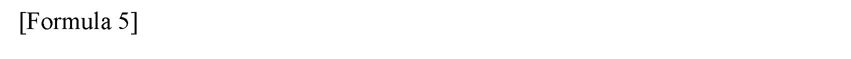 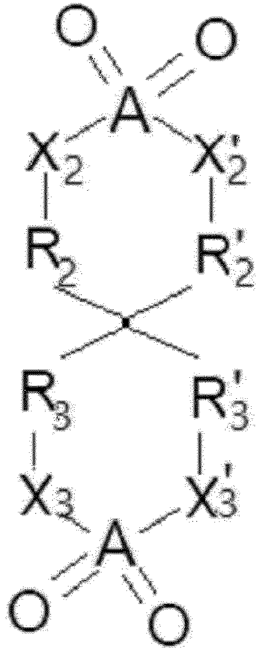 (In Formulas 4 to 5, A is phosphorus (P), sulfur (S), or nitrogen (N); X₁, X₂, X₃, X₁', X₂' and X₃' are independently a bond or oxygen; n is an integer of 0 to 3; and R₁, R₂, R₃, R₁', R₂' and R₃' are independently a bond, substituted or unsubstituted alkylene with 1 to 10 carbon atoms and have at least one carbon.) 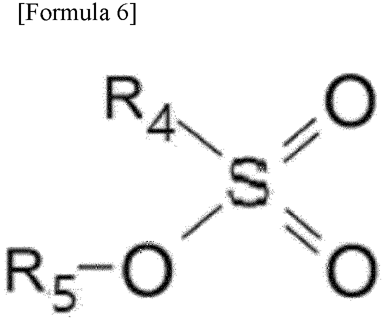 (In Formula 6, R₄ is OR₆ or R₆; R₅ is R₇A; A is 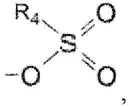 and R₆ and R₇ are independently an alkyl group with 1 to 5 carbon atoms or an aryl group with 6 to 10 carbon atoms.)

As a specific example, the compounds represented by Formulas 3 to 6 have a symmetric linear structure or asymmetric ring structure to stabilize the electron flow within the molecule and enhance molecule rigidity, which is desirable in terms of molecular structure stabilization, chemical stability, ion mobility of the electrolyte, and prevention of side reactions of the electrode active material. In addition, the secondary battery containing the above first additive has a lowered charging resistance, improving battery output, increasing charging recovery capacity at high temperature, and increasing lifespan efficiency. Thus, the first additive is desirable as an electrolyte additive for batteries.

The halogen substituent contained in the compound represented by Formulas 4 and 5 may be, for example, fluorine or iodine, and preferably may be fluorine. Fluorine is the element with the highest electronegativity of 3.98. If halogen substituent is fluorine, the polarity of the compound represented by Formulas 4 and 5 increases. Through this, the ion mobility of the electrolyte, including the compound represented by Formulas 4 and 5 and the organic solvent, may be improved. Also, since the compound forms a hydrogen bond with the electrode active material on the electrode surface, it is possible to prevent side reactions with the electrode active material, which may occur during charging and discharging of the battery, thereby maximizing the effect of improving battery stability and charge/discharge efficiency.

R₁, R₂ and R₃ are preferably independently a phenyl group, a methyl group or an ethyl group, respectively. If R₁, R₂ and R₃ are all phenyl groups, they are chemically stable to become inert, and the molecular structure is simplified, which is most preferable in terms of stability.

In Formula 4 or 5, A₁ and A₂ are independently phosphorus or sulfur, respectively, and in terms of the above-mentioned effects, phosphorus having a relatively large difference in electronegativity with the O element is more preferable. However, if each of X1, X' 1, X2, and X'2 is oxygen, sulfur having a relatively small difference in electronegativity with the O element is preferred.

R₁, R'₁, R₂, R'₂, R₃, R'₃, R₆ and R₇ are independently a straight-chain alkyl group or alkylene group containing no substituent or a halogen substituent with 1 to 5 carbon atoms, respectively. Preferably, they are preferably an alkyl group or an alkylene group containing no substituent or a halogen substituent with 1 or 2 carbon atoms, and more preferably, they may be a methyl group or a methylene group. If R₁, R'₁, R₂, R'₂, R₃, R'₃, R₆ and R₇ are all methyl or methylene groups, they are chemically stable to become inert, and the molecular structure is simplified, which is most desirable in terms of stability.

As a specific example, the first additive may be at least one selected from compounds represented by Formulas 7 to 13 below. 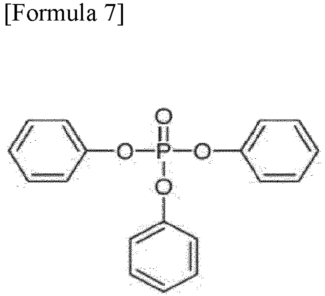 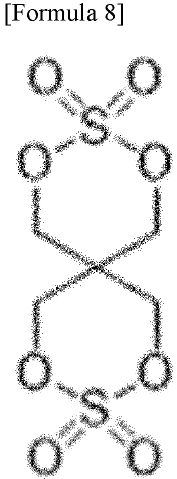 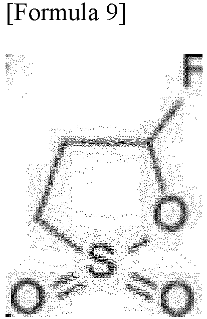 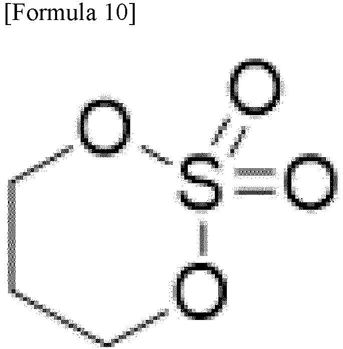 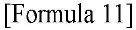 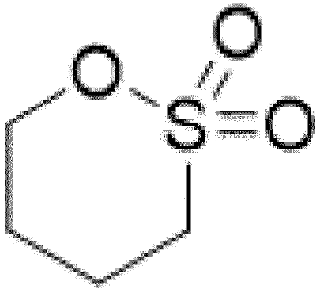 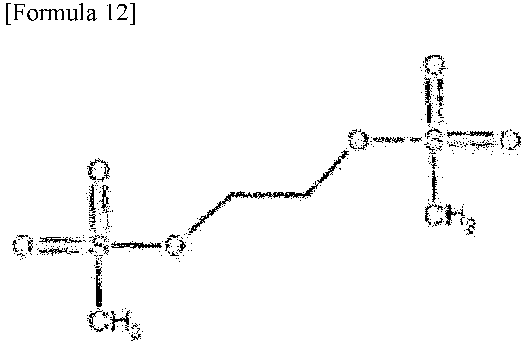 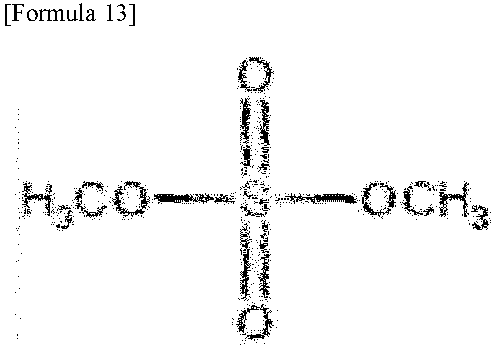 (In Formulas 7 to 13, a solid line is a bond; a point where a bond meets a bond is carbon when a separate element is not described; and the number of hydrogens satisfying the atom value of the carbon is omitted.)

The electrolyte of the present disclosure may contain the first additive in an amount of 15 weight% or less, preferably 0.01 to 10 weight%, based on a total of 100 weight% of the electrolyte. Within this range, the decomposition effect of electrolytes is suppressed, which has the advantage of improving battery life characteristics and cycle characteristics.

For example, the second additive may have an atomic group represented by Formula 1 above. In this case, the stability of the electrolyte may be further improved by adsorbing to the metal surface of the electrode and suppressing side reactions between the electrode and the electrolyte, thereby further improving the stability of the electrolyte and thus improving the cycle characteristics, stability, and lifespan of the battery. 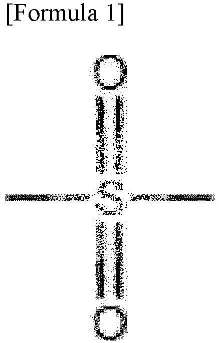 (In Formula 1, a solid line is a bond.)

The term "atomic group" used in this description refers to a covalently bonded polyatom ion, unless otherwise specified.

The second additive may be a compound containing a pair of at least one cation selected from the group consisting of alkali metal ions (excluding lithium) and an anion represented by Formula 14 below. 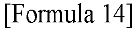 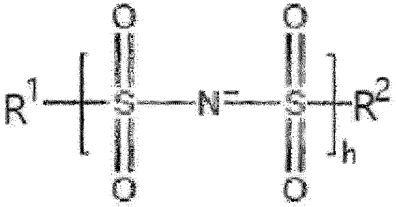 (In Formula 14, R¹ and R² are independently hydrogen, halogen, or a straight-chain or branched alkyl group containing a halogen substituent with 1 to 7 carbon atoms, and h is an integer of 1 to 10.)

If the electrolyte additive containing a compound represented by Formula 14 is added to the electrolyte of a secondary battery, electrons are localized toward the N element due to the difference in electronegativity between the N element and the S element directly connected to the N element. As a result, the S element becomes electron-poor (e-poor, δ+), which induces an oxidation reaction in the electrolyte containing lithium ions, forming a stable film on the electrode, for example, the positive electrode (cathode), and at the same time improving ionic conductivity due to the halogen substituent substituted to the terminal.

At this time, the decomposition of the electrolyte may be prevented due to the stability of the film, and this may improve cycle characteristics. In particular, since the electrolyte is not decomposed at high temperature, compared to the conventional electrode film that decomposes at high temperature to deteriorate high temperature storage, there is an excellent effect of greatly improving high temperature storage. In addition, an increase in resistance is prevented, which has the effect of improving charging efficiency and output, and gas generation due to chemical reactions inside the battery is also suppressed, thereby improving the safety of the battery.

Specifically, gas generation inside the battery is mainly caused by the decomposition of electrolyte components, especially carbonate-based solvents, on the surface of the positive/negative electrode, which may easily deteriorate the positive and negative electrode protective films or may also be further accelerated by oxygen radicals generated from the positive electrode. This material may suppress direct decomposition of the solvent by forming a highly stable protective film composed of N, S, O, and F components, and may prevent elution of transition metal ions from the positive electrode due to positive electrode deterioration caused by the metal ion coordination effect. Ultimately, it is possible to prevent the escape of oxygen elements that form the framework of the positive electrode.

In addition, capacity retention is improved by preventing collapse of the electrode active material structure of the positive electrode and the negative electrode at high temperature, which has the effect of extending lifespan.

The second additive may be a compound containing a pair of a cation of cesium or sodium and at least one anion selected from the group represented by Formulas 15 to 17 below. If an electrolyte containing the second additive is used to construct a lithium secondary battery, the terminal group of the corresponding compound has the effect of suppressing the decomposition of the electrolyte, thereby reducing the rate of increase in internal resistance, gas generation, and thickness increase rate, which gives the effect of extending the lifespan of the battery. 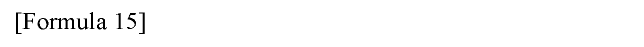 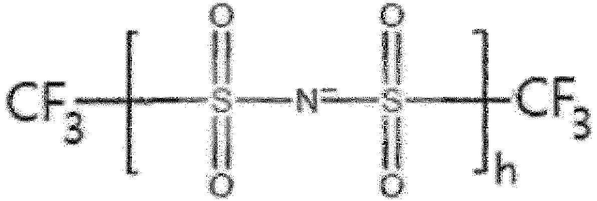 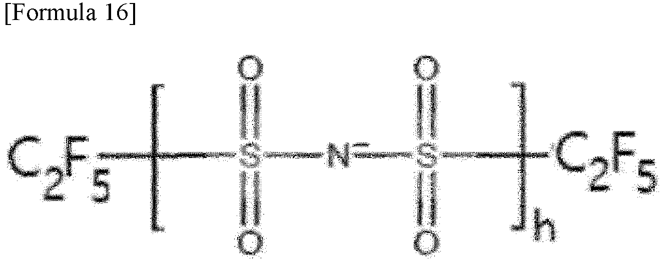 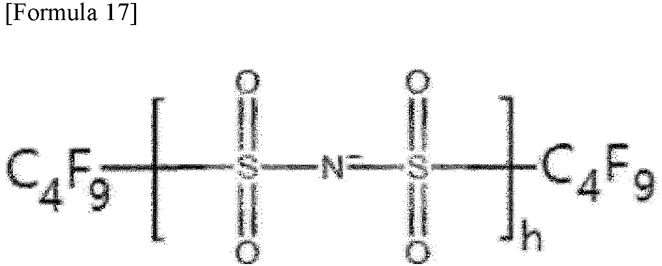 (In Formulas 15 to 17, h is an integer of 1 to 10.)

As a specific example, when the cation is cesium and the anion has a structure represented by Formula 16, there is a great advantage in improving battery performance because the electron flow within the molecule may be stabilized as a symmetrical linear structure.

The compound represented by Formula 14 is preferably symmetrical around N, and in this case, the symmetrical linear structure stabilizes the electron flow within the molecule, which gives an advantage of significantly improving battery performance.

The electrolyte of the present disclosure may contain the second additive in an amount of 0.1 to 10 weight%, preferably 1 to 5 weight%, based on a total of 100 weight% of the electrolyte. Within this range, the decomposition effect of the electrolyte is suppressed, which has the advantage of improving battery life characteristics and cycle characteristics.

The electrolyte of the present disclosure may maximize the effect of suppressing side reactions of the electrolyte by using a combination of the first additive and the second additive. If a lithium secondary battery is configured to include the electrolyte, the amount of gas generated when the lithium secondary battery is left along at high temperature is low, which has the effect of reducing the rate of increase in internal resistance and ultimately improving the lifespan characteristics of the battery.

The first additive and the second additive may give the effect of reducing discharge resistance when being left alone at high temperature, if they are included in a weight ratio of 1 :0.5 to 2.5 (first additive: second additive), or 1 :0.5 to 2 based on a total of 100 weight% of the electrolyte.

The non-aqueous solvent that can be included in the electrolyte of the present disclosure is not particularly limited as long as it may minimize decomposition due to oxidation reactions during the charging and discharging process of the battery and may exhibit the desired properties together with the additive. For example, the non-aqueous solvent may be carbonate-based organic solvents, propionate-based organic solvents, or the like. These materials may be used individually, or two or more types may be used in combination.

For example, 0 to 40 volume% of ethylene carbonate, and 5 to 80 volume% of at least one selected from the group consisting of ethylmethyl carbonate, diethyl carbonate, dimethyl carbonate, ethyl propionate, propyl propionate, and propylene carbonate may be mixed and used as the non-aqueous solvent.

Among the above non-aqueous solvents, the carbonate-based organic solvent may be, for example, at least one selected from the group consisting of ethylene carbonate (EC), propylene carbonate (PC), butylene carbonate (BC), vinylene carbonate (VC), dimethyl carbonate (DMC), diethyl carbonate (DEC), dipropyl carbonate (DPC), ethylmethyl carbonate (EMC), methylethyl carbonate (MEC), fluoroethylene carbonate (FEC), methylpropyl carbonate (MPC), and ethylpropyl carbonate (EPC).

Among the carbonate-based organic solvents, it may be more preferable to use a mixture of a carbonate-based organic solvent with a high dielectric constant that may improve the charging and discharging performance of the battery and a carbonate-based organic solvent with a low viscosity that may appropriately control the viscosity of the high dielectric constant organic solvent.

Specifically, a high dielectric constant organic solvent selected from the group consisting of ethylene carbonate, propylene carbonate, and mixtures thereof, and a low viscosity organic solvent selected from the group consisting of ethylmethyl carbonate, dimethyl carbonate, diethyl carbonate, and mixtures thereof may be mixed and used.

Preferably, it is better to use a mixture of the high dielectric constant organic solvent and the low viscosity organic solvent at a volume ratio of 2:8 to 8:2, and more specifically, ethylene carbonate or propylene carbonate; ethyl methyl carbonate; and dimethyl carbonate or diethyl carbonate may be mixed and used at a volume ratio of 5:1:1 to 2:5:3, for example 3:5:2.

As a specific example, the carbonate-based organic solvent may include ethylene carbonate (EC), diethyl carbonate (DEC), and ethylmethyl carbonate (EMC), and 10 to 40 weight%, or 15 to 35 weight%, or 20 to 30 weight%, or 22 to 28 weight% of ethylene carbonate; 15 to 45 weight%, or 20 to 40 weight%, or 25 to 35 weight%, or 27 to 33 weight% of diethyl carbonate; and 30 to 60 weight%, or 35 to 55 weight%, or 40 to 50 weight%, or 42 to 48 weight% of ethyl methyl carbonate may be mixed and used.

In addition, among the non-aqueous solvents, the propionate-based organic solvent may include propionate, methyl propionate, or the like, but is not limited thereto.

For example, the organic solvent may be used as the remaining amount of electrolyte after subtracting the content of components other than the organic solvent from the electrolyte.

If the organic solvent contains moisture, lithium ions in the electrolyte may be hydrolyzed, so the moisture in the organic solvent is preferably controlled to 150 ppm or less, preferably 100 ppm or less.

Lithium salts that may be included in the electrolyte of the present disclosure may be, for example, at least one selected from the group consisting of LiPF₆, LiClO₄, LiAsF₆, LiBF₄, LiBF₆, LiSbF₆, LiAl0₄, LiAlCl₄, LiClO₄, LiCF₃SO₃, LiC₄F₉SO₃, LiN(C₂F₅SO₃)₂, LiN(C₂F₅SO₂)₂, LiN(CF₃SO₂)₂, LiN(CaF₂ₐ₊₁SO₂) (C_{b}F_{2b+1}SO₂) (where a and b are natural numbers), LiCl, LiI, and LiB(C₂O₄)₂, among which LiPF₆ is preferred. Here, a and b may be integers from 1 to 4, for example.

If the lithium salt is dissolved in the electrolyte, the lithium salt may function as a source of lithium ions within the lithium secondary battery and promote the movement of lithium ions between the positive electrode and the negative electrode. Accordingly, the lithium salt is preferably included in the electrolyte at a concentration of approximately 0.6 to 2M. If the concentration of the lithium salt is less than 0.6M, the conductivity of the electrolyte may decrease and electrolyte performance may deteriorate. If the concentration of the lithium salt exceeds 2M, the viscosity of the electrolyte may increase and thus the mobility of lithium ions may decrease.

Considering the conductivity of the electrolyte and the mobility of lithium ions, the lithium salt may be included in the electrolyte at a concentration of 0.5 to 1.5M (mol/L), preferably at a concentration of 0.7 to 1.3M, more preferably at a concentration of 0.8 to 1.1M. Within this range, the conductivity of the electrolyte is high, resulting in excellent electrolyte performance, and the low viscosity of the electrolyte results in excellent mobility of lithium ions.

For example, the electrolyte may have a lithium ion conductivity of 0.3 S/m or more, or 0.3 to 10 S/m under conditions of 25°C, and within that range, the cycle life characteristics of the lithium secondary battery may be further improved.

In addition to the electrolyte components, the electrolyte may further include electrolyte additives that may be generally used in electrolytes for the purposes of improving battery life characteristics, suppressing battery capacity reduction, and improving battery discharge capacity.

For example, the above electrolyte additive may be at least one selected from the group consisting of boron compounds, phosphorus compounds, sulfur compounds, and nitrogen-based compounds (hereinafter, also referred to as a 'third additive').

For example, the third additive may be at least one selected from compounds represented by Formulas 18 to 25 below, and by including the third additive, the decomposition reaction of the electrolyte may be more effectively suppressed. 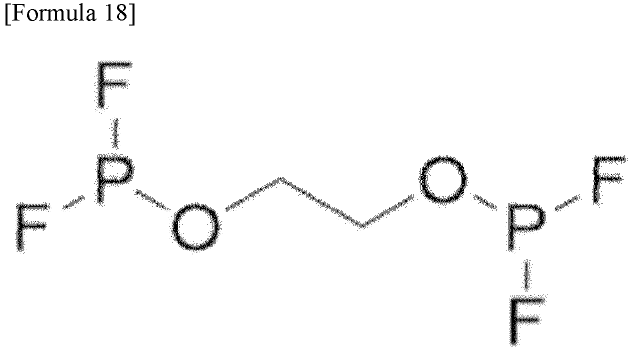 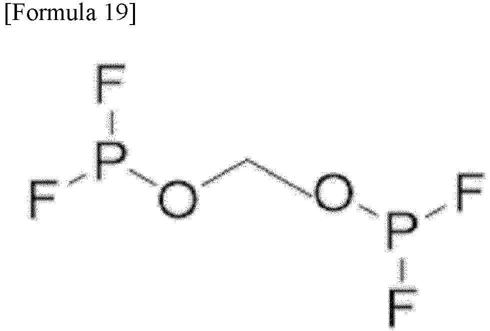 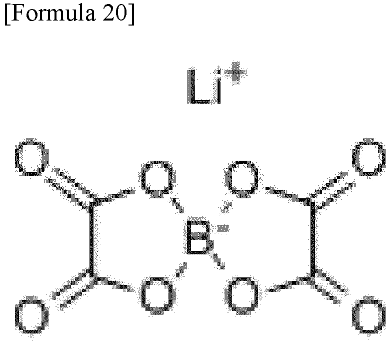 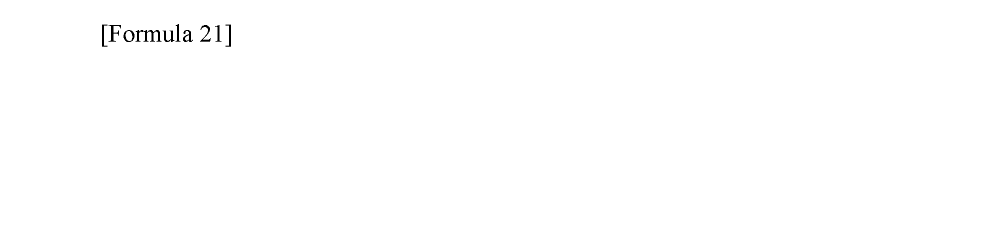 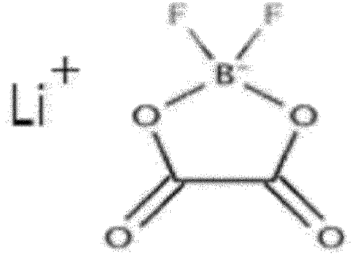 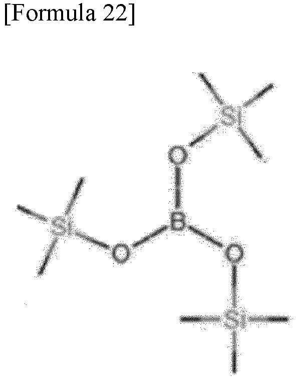 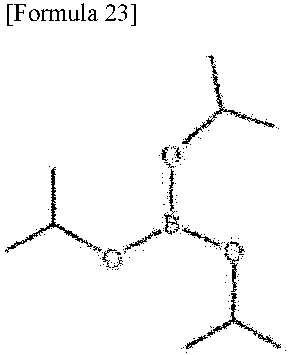 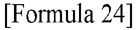 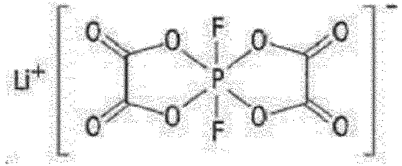 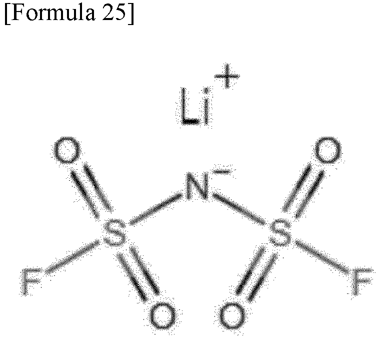 (In Formulas 18 to 25, a solid line is a bond, and unless a separate element is described, the point where a bond meets a bond is carbon, and the number of hydrogens satisfying the atom value of the carbon is omitted.)

The electrolyte of the present disclosure may contain the third additive in an amount of 10 weight% or less, preferably 1 to 5 weight%, based on a total of 100 weight% of the electrolyte. Within this range, the electrolyte decomposition effect is suppressed, which has the advantage of improving life characteristics and cycle characteristics of the battery.

In addition, the electrolyte additive may include, for example, metal fluoride, and if the metal fluoride is further included as the electrolyte additive, the influence of acid generated around the positive electrode active material is reduced, and the reaction between the positive electrode active material and the electrolyte is reduced, thereby solving the phenomenon of rapid decrease in battery capacity.

The metal fluorides may be specifically at least one selected from the group consisting of LiF, RbF, TiF, AgF, AgF₂, BaF₂, CaF₂, CdF₂, FeF₂, HgF₂, Hg₂F₂, MnF₂, NiF₂, PbF₂, SnF₂, SrF₂, XeF₂, ZnF₂, AlF₃, BF₃, BiF₃, CeF₃, CrF₃, DyF₃, EuF₃, GaF₃, GdF₃, FeF₃, HoF₃, InF₃, LaF₃, LuF₃, MnF₃, NdF₃, PrF₃, SbF₃, ScF₃, SmF₃, TbF₃, TiF₃, TmF₃, YF₃, YbF₃, TIF₃, CeF₄, GeF₄, HfF₄, SiF₄, SnF₄, TiF₄, VF₄, ZrF₄, NbF₅, SbF₅, TaF₅, BiF₅, MoF₆, ReF₆, SF₆, WF₆, CoF₂, CoF₃, CrF₂, CsF, ErF₃, PF₃, PbF₃, PbF₄, ThF₄, TaF₅, and SeF₆.

For example, the metal fluoride may be included in an amount of 0. 1 to 10 weight%, or 0.2 to 5 weight%, based on the total weight of the electrolyte, and within this range, the cycle life characteristics of the lithium secondary battery may be further improved.

The electrolyte according to the present disclosure with the above composition suppresses the decomposition reaction of the electrolyte in a wide temperature range of -20°C to 60°C, resulting in reducing the amount of gas generation and the rate of increase in internal resistance, so it is possible to provide an electrolyte secondary battery with high stability and reliability. In addition, the structure of the battery itself is the same as that of a general electrolyte secondary battery, so the secondary battery of the present disclosure is advantageous for manufacture and mass-production.

The lithium secondary battery according to the present disclosure may include a positive electrode; a negative electrode; a separator provided between the positive electrode and the negative electrode; and an electrolyte. The electrolyte may include the above-described electrolyte, and the positive electrode and negative electrode may include a positive electrode active material and a negative electrode active material, respectively.

The positive electrode may be manufactured, for example, by mixing a positive electrode active material, a binder, and optionally a conductive agent to prepare a composition for forming a positive electrode active material layer, and then applying the composition to a positive electrode current collector such as aluminum foil.

The positive electrode active material may be, for example, lithium composite metal oxide and lithium olivine-type phosphate typically used in lithium secondary batteries. Preferably, the positive electrode active material may include at least one metal selected from the group consisting of cobalt, manganese, nickel, and iron, and more preferably, may use NCM (lithium·nickel·cobalt oxide).

As a specific example, the positive electrode active material may be a lithium composite metal oxide in the form of Li[NiₓCo_{1-x-y}Mn_{y}]O₂ (where 0<x<0.5, 0<y<0.5), but is not limited thereto. The variables x and y of the formula Li[NiₓCo_{1-x-y}Mn_{y}]O₂ of the lithium composite metal oxide may be, for example, 0.0001<x<0.5, 0.0001<y<0.5, or 0.001<x<0.3, 0.001<y<0.3.

As another example, the positive electrode active material may be a compound allowing reversible intercalation and de-intercalation of lithium (lithiated intercalation compound). Among the compounds, the positive electrode active material is preferably at least one selected from the group consisting of LiCoO₂, LiMnO₂, LiMn₂O₄, LiNiO₂, LiNiₓMn₍₁₋ₓ₎O₂ (where 0<x<1), and LiMlₓM2_{y}O₂ (where 0≤x≤1, 0≤y≤1, 0≤x+y≤1, M1 and M2 are independently selected from the group consisting of Al, Sr, Mg, and La) in that they may improve the capacity characteristics and stability of the battery.

In addition, among the positive electrode active materials, the lithium olivine-type phosphate may preferably include at least one selected from iron, cobalt, nickel, and manganese, and specifically may include LiFePO₄, LiCoPO₄, and LiMnPO₄. In addition, a compound obtained by substituting some metals of the lithium olivine phosphate with other metals may also be used.

The negative electrode may be manufactured, for example, by mixing a negative electrode active material, a binder, and optionally a conductive agent to prepare a composition for forming a negative electrode active material layer, and then applying the composition to a negative electrode current collector such as copper foil.

For example, the negative electrode active material may be a compound allowing reversible intercalation and de-intercalation of lithium.

The negative electrode active material may include, for example, at least one selected from the group consisting of tin, tin compound, silicon, silicon compound, lithium titanate, crystalline carbon, amorphous carbon, artificial graphite, and natural graphite.

In this description, the tin compound or the silicon compound is a compound in which tin or silicon is combined with one or more other chemical elements, respectively.

In addition, in addition to the carbonaceous materials including crystalline carbon, amorphous carbon, graphite, or the like, metallic compounds that may be alloyed with lithium, or composites including metallic compounds and carbonaceous materials may also be used as the negative electrode active material. For example, graphite may be used.

The metal that may be alloyed with lithium may be, for example, at least one of Si, Al, Sn, Pb, Zn, Bi, In, Mg, Ga, Cd, Si alloy, Sn alloy, or Al alloy. Also, a thin film of metallic lithium may be used as the negative electrode active material.

The negative electrode active material may be any one or more selected from the group consisting of crystalline carbon, amorphous carbon, carbon composite, lithium metal, and alloy containing lithium in terms of high stability.

For example, a battery assembly may be prepared by placing a separator between the positive electrode and the negative electrode described above, inserting them into a cell, then injecting an electrolyte into the cell, and sealing the cell. At this time, it is obvious that the lithium secondary battery including the electrolyte, the positive electrode, the negative electrode, and the separator as described above may be formed as, for example, a unit cell having a structure of positive electrode/separator/negative electrode, and a bi-cell having a structure of positive electrode/separator/negative electrode/separator/positive electrode, or a stacked cell having a structure where the unit cell structure is formed repeatedly.

By adding the first additive and second additive to improve battery performance, the secondary battery according to an embodiment of the present disclosure have the effects of improving battery characteristics such as battery discharge resistance measured by the HPPC (Hybrid Pulse Power Characterization) method, output characteristics, and capacity recovery characteristics and lifespan characteristics at high temperature of 60°C or higher.

By adding a performance improver in addition to the first additive and second additive added to the electrolyte, the secondary battery according to another embodiment of the present disclosure has the effect of improving battery characteristics such as battery discharge resistance measured by the HPPC (Hybrid Pulse Power Characterization) method, output characteristics, and capacity recovery characteristics and lifespan characteristics at high temperature of 60°C or higher.

Specifically, the secondary battery of the present disclosure may have an HPPC discharge resistance value measured at 60 °C of 28 mΩ or less, preferably 25 to 28 mQ.

In this description, the HPPC discharge resistance value may be measured by the method specified in the document "Battery test manual for plug-in hybrid electric vehicles" (2010, Idaho National Laboratory for the U.S. Department of Energy), and it is an important indicator that indicates the characteristics of the battery, such as battery output. In addition, the discharge resistance is a resistance value measured when discharging a battery, and may provide improved output performance within the above range. The lower the discharge resistance, the less the energy loss, the faster charging the speed, resulting in improved battery output. The secondary battery of the present disclosure has excellent charging speed and output since the HPPC discharge resistance value is reduced by up to 23.6%, so it is suitable for use as a vehicle battery, for example.

Specifically, the secondary battery of the present disclosure may have a recovery capacity measured at 60°C of 585 mAh or more, preferably 585.5 to 606.5 mAh.

In this description, the recovery capacity refers to the capacity preservation characteristics of a battery that has been left alone for a long time, and is obtained by measuring the discharged electric capacity when the battery left alone for a long time is discharged and the discharged electric capacity when the discharged battery is charged again and discharged again to the discharge end voltage, and then comparing both capacity values. The higher the recovery capacity, the smaller the amount of natural discharge amount due to battery preservation (storage), which means that long-term storage of the battery is possible. In particular, the higher the storage temperature of the battery, the faster the natural discharge speed, so the recovery capacity at high temperature is a very important characteristic in the battery for a vehicle. If the electrolyte additive of the present disclosure is added to the electrolyte for a battery, the recovery capacity is improved as above, which has the effect of enabling longer storage with a single charge.

Also, the secondary battery may have a thickness measured at 60°C of 2.6 mm or less, preferably 2.4 to 2.6 mm.

In addition, the secondary battery may have a thickness increase rate of 13.04% or less, preferably 4.35 to 13.04%, at 60°C as calculated by Equation 1 below.Thickness increase rate (%) = {(thickness after storage at high temperature - initial thickness) / initial thickness) X 100

In this description, the thickness increase rate refers to the swelling characteristic caused by gas generation inside the battery, and is obtained by measuring the initial thickness of a pouch cell and the thickness after the pouch cell is left alone at high temperature and then comparing the difference between both values. If a stable film is formed on the positive electrode and the negative electrode, decomposition of electrolyte components may be suppressed. Thus, as the thickness increase rate is lower, it is possible to provide stability during repeated charging and discharging of the battery, which has the effect of providing improved battery lifespan.

Also, the secondary battery may have a coulombic efficiency of 99.5% or more, preferably 99.5 to 99.9%, as measured by Equation 2 below. Coulombic efficiency (%) = (discharge capacity at 300 cycles / charge capacity at 300 cycles) X 100

In this description, the coulombic efficiency is calculated based on the charge capacity and the discharge capacity at 300 cycles, and has the effect of improving charging and discharging efficiency.

In the secondary battery, the cell thickness may increase by less than 4 mm due to the generation of CO₂ gas and H₂ gas due to electrolyte decomposition and SEI film formation during 300 cycle charge and discharge.

Therefore, if the battery of the present disclosure is used as a vehicle battery, the output that is important depending on the size of the vehicle is improved, and the performance and lifespan at low and high temperatures that are environmental issues of the vehicle due to climate change and the vehicle being directly exposed to sunlight while driving or parking are improved, thereby enabling excellent performance as a vehicle battery.

The lithium secondary battery according to this description may be classified into a lithium ion battery, a lithium ion polymer battery, and a lithium polymer battery depending on the type of separator and electrolyte used, and may be classified into cylindrical, square, coin-type, or pouch-type batteries depending on shape, and may be classified into bulk-type and thin film-type batteries depending on size. The electrolyte according to an embodiment of the present disclosure is particularly excellent for application to lithium ion batteries, aluminum laminated batteries, and lithium polymer batteries.

The lithium secondary battery containing the electrolyte according to the present disclosure may improve life characteristics, increase internal resistance, and reduce thickness increase rate and gas generation rate, especially at high temperatures of 45°C or higher, for example, 45°C to 60°C. This, if the battery of the present disclosure is used as a vehicle battery, the output that is important depending on the size of the vehicle is improved, and the performance and lifespan at low and high temperatures that are environmental issues of the vehicle due to climate change and the vehicle being directly exposed to sunlight while driving or parking are improved, thereby enabling excellent performance as a vehicle battery.

In other words, when the electrolyte additive according to the embodiments of the present disclosure and the electrolyte containing the electrolyte additive are applied to the secondary battery, the charging resistance, output, recovery capacity and life efficiency are improved, so the secondary battery is suitable for use as a secondary battery for vehicles. In addition, the secondary battery may be useful for portable devices such as mobile phones, laptop computers, digital cameras, and camcorders, electric vehicles such as hybrid electric vehicles (HEV) and plug-in hybrid electric vehicles (plug-in HEV, PHEV), and mid- to large-sized energy storage systems.

Hereinafter, preferred examples are presented to help understand the present disclosure. However, the following examples are merely illustrative of the present disclosure, and various changes and modifications are possible within the scope and technical idea of the present disclosure, and it is obvious that such changes and modifications fall within the scope of the appended claims.

### [Example: Preparation of an electrolyte for a battery]

### Example 1-1, and Comparative Examples 1-1 to 1-3

LiPF₆ as lithium salt was dissolved in a carbonate-based mixed solvent with a volume ratio of EC:EMC:DMC = 2:4:4 to a concentration of 1.15M to prepare an organic solvent, and a compound represented by Formula 1a below (also indicated as Formula 18) to serve as a first electrolyte additive, and a compound represented by Formula 2a below (also represented by Formula 14) where h is 1 and the cation is cesium or a compound represented by Formula 2a below where h is 1 and the cation is sodium to serve as a second electrolyte additive were added in amounts shown in Table 1 below to prepare an electrolyte for a battery.

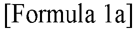

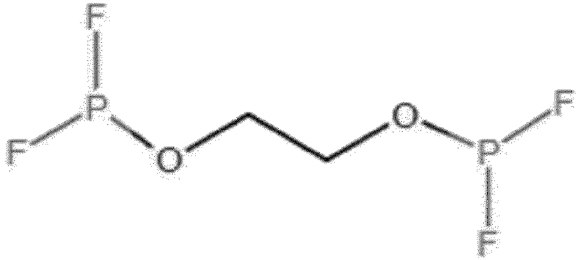

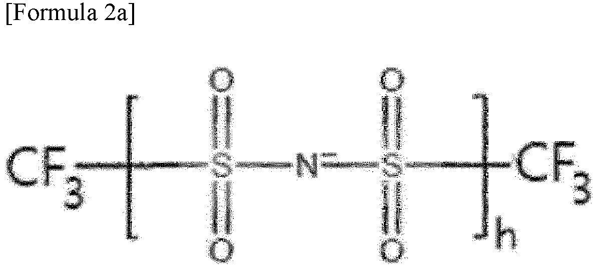

**Table 1**

| | Type of first additive | Content of first additive (wt%) | Type of second additive | Content of second additive (wt%) |
|---|---|---|---|---|
| Example 1-1 | Formula 1a | 10 | Formula 2a+cesium | 5 |
| Comparative Example 1-1 | Formula 1a | 10 | - | - |
| Comparative Example 1-2 | Formula 1a | 10 | Formula 2a+sodium | 5 |
| Comparative Example 1-3 | - | - | Formula 2a+cesium | 5 |

### Examples 2-1 to 2-3, and Comparative Examples 2-1 to 2-10

LiPF₆ as lithium salt was dissolved in a carbonate-based mixed solvent with a volume ratio of EC:EMC:DMC = 2:4:4 to a concentration of 1.15M to prepare an organic solvent, and a compound represented by Formula 1b below (also indicated as Formula 20), a compound represented by Formula 1c below (also represented by Formula 21), or a compound represented by Formula 1d below (also represented by Formula 22) to serve as a first electrolyte additive, and a compound represented by Formula 2a below where h is 1 and the cation is cesium or a compound represented by Formula 2a below where h is 1 and the cation is sodium to serve as a second electrolyte additive were added in amounts shown in Table 2 below to prepare an electrolyte for a battery.

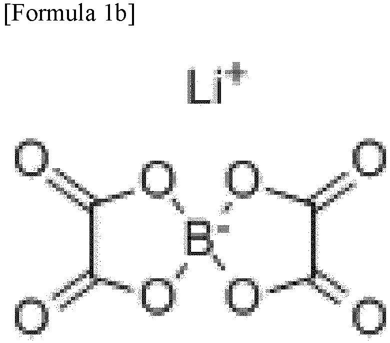

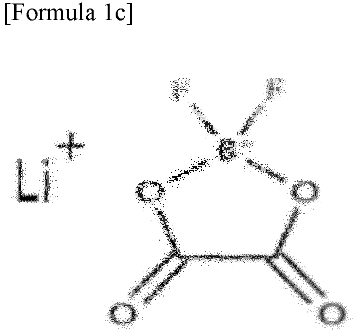

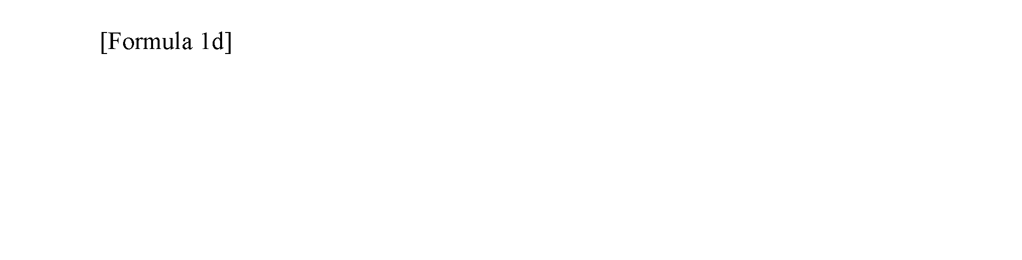

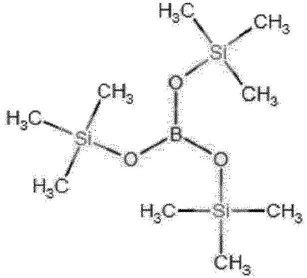

**Table 2**

| | Type of first additive | Content of first additive (wt%) | Type of second additive | Content of second additive (wt%) |
|---|---|---|---|---|
| Example 2-1 | Formula 1b | 10 | Formula 2a+cesium | 5 |
| Example 2-2 | Formula 1c | 10 | Formula 2a+cesium | 5 |
| Example 2-3 | Formula 1d | 10 | Formula 2a+cesium | 5 |
| Comparative Example 2-1 | Formula 1b | 5 | Formula 2a+cesium | 10 |
| Comparative Example 2-2 | Formula 1b | 5 | Formula 2a+sodium | 5 |
| Comparative Example 2-3 | - | - | Formula 2a+cesium | 5 |
| Comparative Example 2-4 | Formula 1b | 10 | - | - |
| Comparative Example 2-5 | Formula 1c | 5 | Formula 2a+cesium | 10 |
| Comparative Example 2-6 | Formula 1c | 10 | Formula 2a+sodium | 5 |
| Comparative Example 2-7 | Formula 1c | 10 | - | - |
| Comparative Example 2-8 | Formula 1d | 5 | Formula 2a+cesium | 10 |
| Comparative Example 2-9 | Formula 1d | 10 | Formula 2a+sodium | 5 |
| Comparative Example 2-10 | Formula 1d | 10 | - | - |

### Example 3-1, and Comparative Examples 3-1 and 3-2

LiPF₆ as lithium salt was dissolved in a carbonate-based mixed solvent with a volume ratio of EC:EMC:DMC = 2:4:4 to a concentration of 1.15M to prepare an organic solvent, and a compound represented by Formula 1e below (also represented by Formula 23) to serve as a first electrolyte additive, and a compound represented by Formula 2a below where h is 1 and the cation is cesium or a compound represented by Formula 2a below where h is 1 and the cation is sodium to serve as a second electrolyte additive were added in amounts shown in Table 3 below to prepare an electrolyte for a battery.

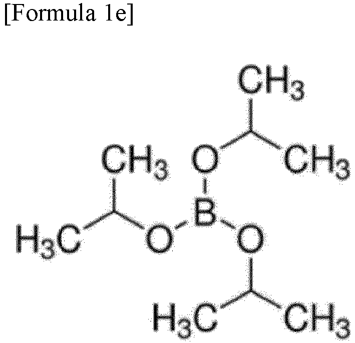

**Table 3**

| | Type of first additive | Content of first additive (wt%) | Type of second additive | Content of second additive (wt%) |
|---|---|---|---|---|
| Example 3-1 | Formula 1e | 10 | Formula 2a+cesium | 5 |
| Comparative Example 3-1 | Formula 1e | 10 | - | - |
| Comparative Example 3-2 | Formula 1e | 10 | Formula 2a+sodium | 5 |

### Example 4-1, and Comparative Examples 4-1 to 4-3

LiPF₆ as lithium salt was dissolved in a carbonate-based mixed solvent with a volume ratio of EC:EMC:DMC = 2:4:4 to a concentration of 1.15M to prepare an organic solvent, and a compound represented by Formula 1f below (also indicated as Formula 24) to serve as a first electrolyte additive, and a compound represented by Formula 2a below where h is 1 and the cation is cesium or a compound represented by Formula 2a below where h is 1 and the cation is sodium to serve as a second electrolyte additive were added in amounts shown in Table 4 below to prepare an electrolyte for a battery.

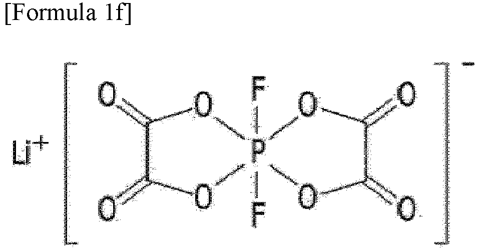

**Table 4**

| | Type of first additive | Content of first additive (wt%) | Type of second additive | Content of second additive (wt%) |
|---|---|---|---|---|
| Example 4-1 | Formula 1f | 10 | Formula 2a+cesium | 5 |
| Comparative Example 4-1 | Formula 1f | 15 | Formula 2a+cesium | 5 |
| Comparative Example 4-2 | Formula 1f | 10 | Formula 2a+sodium | 5 |
| Comparative Example 4-3 | Formula 1f | 10 | - | - |

### Examples 5-1 to 5-7, and Comparative Examples 5-1 to 5-10

LiPF₆ as lithium salt was dissolved in a carbonate-based mixed solvent with a volume ratio of EC:EMC:DMC = 2:4:4 to a concentration of 1.15M to prepare an organic solvent, and a compound represented by Formulas 1g to 1m below (also indicated as Formulas 7, 8, 13, 14, 9, 10, 11, respectively) to serve as a first electrolyte additive, and a compound represented by Formula 2a below where h is 1 and the cation is cesium or a compound represented by Formula 2a below where h is 1 and the cation is sodium to serve as a second electrolyte additive were added in amounts shown in Table 5 below to prepare an electrolyte for a battery.

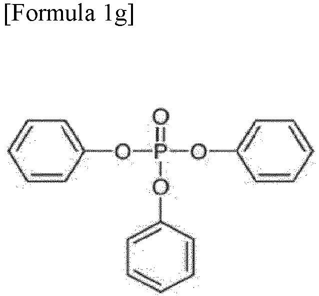

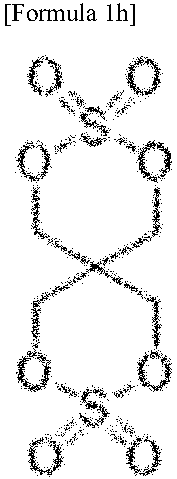

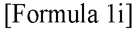

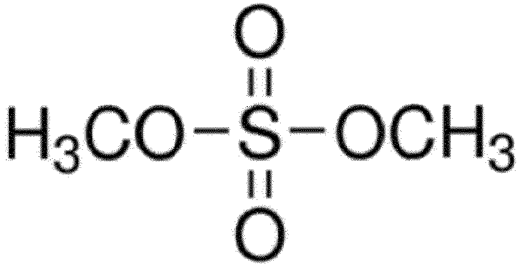

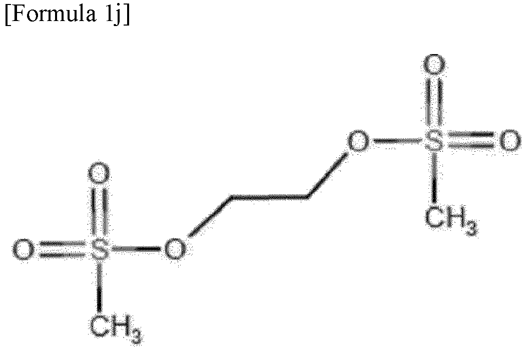

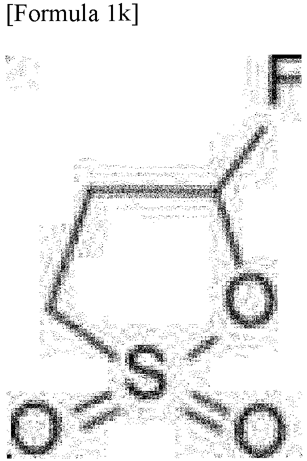

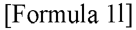

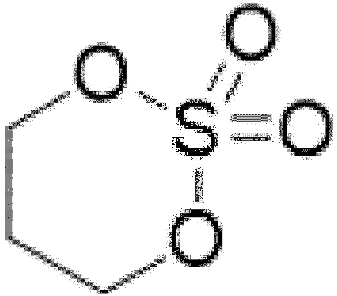

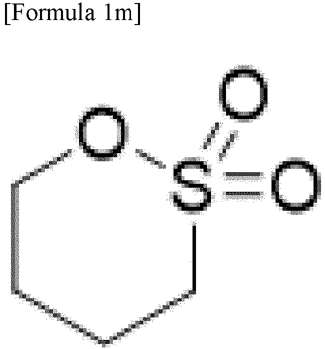

**Table 5**

| | Type of first additive | Content of first additive (wt%) | Type of second additive | Content of second additive (wt%) |
|---|---|---|---|---|
| Example 5-1 | Formula 1g | 10 | Formula 2a+cesium | 5 |
| Example 5-2 | Formula 1h | 10 | Formula 2a+cesium | 5 |
| Example 5-3 | Formula 1i | 10 | Formula 2a+cesium | 5 |
| Example 5-4 | Formula 1j | 10 | Formula 2a+cesium | 5 |
| Example 5-5 | Formula 1k | 10 | Formula 2a+cesium | 5 |
| Example 5-6 | Formula 1l | 10 | Formula 2a+cesium | 5 |
| Example 5-7 | Formula 1m | 10 | Formula 2a+cesium | 5 |
| Comparative Example 5-1 | Formula 1g | 5 | Formula 2a+sodium | 5 |
| Comparative Example 5-2 | Formula 1g | 10 | - | - |
| Comparative Example 5-3 | Formula 1h | 10 | - | - |
| Comparative Example 5-4 | Formula 1i | 10 | - | - |
| Comparative Example 5-5 | Formula 1j | 10 | - | - |
| Comparative Example 5-6 | Formula 1k | 10 | Formula 2a+sodium | 10 |
| Comparative Example 5-7 | Formula 1k | 10 | - | - |
| Comparative Example 5-8 | Formula 1l | 10 | Formula 2a+sodium | 5 |
| Comparative Example 5-9 | Formula 1l | 10 | - | - |
| Comparative Example 5-10 | Formula 1m | 10 | - | - |

### Example 6-1, and Comparative Examples 6-1 to 6-3

LiPF₆ as lithium salt was dissolved in a carbonate-based mixed solvent with a volume ratio of EC:EMC:DMC = 2:4:4 to a concentration of 1.15M to prepare an organic solvent, and a compound represented by Formula 1n below (also indicated as Formula 20) to serve as a first electrolyte additive, and a compound represented by Formula 2a below where h is 1 and the cation is cesium or a compound represented by Formula 2a below where h is 1 and the cation is sodium to serve as a second electrolyte additive were added in amounts shown in Table 6 below to prepare an electrolyte for a battery.

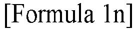

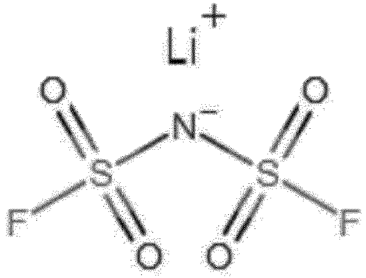

**Table 6**

| | Type of first additive | Content of first additive (wt%) | Type of second additive | Content of second additive (wt%) |
|---|---|---|---|---|
| Example 6-1 | Formula 1n | 10 | Formula 2a+cesium | 5 |
| Comparative Example 6-1 | Formula 1n | 15 | Formula 2a+cesium | 5 |
| Comparative Example 6-2 | Formula 1n | 10 | Formula 2a+sodium | 5 |
| Comparative Example 6-3 | Formula 1n | 10 | - | - |

### Preparation of a battery

100 parts by weight of positive electrode mixture containing 92 weight% of Li(Ni_{0.8}Co_{0.1}Mn_{0.1})O₂ as a positive electrode active material, 4 weight% of carbon black as a conductive agent, and 4 weight% of polyvinylidene fluoride (PVdF) as a binder was added to 100 parts by weight of N-methyl-2-pyrrolidone (NMP) as a solvent to prepare a positive electrode mixture slurry. The positive electrode mixture slurry was applied to an aluminum (Al) thin film that is a positive electrode current collector with a thickness of about 20 µm and dried, and then roll-pressing was performed thereto to manufacture a positive electrode.

In addition, 100 parts by weight of negative electrode mixture containing 96 weight% of carbon powder where artificial graphite and natural graphite were mixed as a negative electrode active material, 3 weight% of PVdF as a binder, and 1 weight% of carbon black as a conductive agent was added to 100 parts by weight of NMP as a solvent to prepare a negative electrode mixture slurry. The negative electrode mixture slurry was applied to a copper (Cu) thin film that is a negative electrode current collector with a thickness of 10 µm and dried, and then roll-pressing was performed thereto to manufacture a negative electrode.

A pouch-type was manufactured in a common manner using the prepared positive electrode and negative electrode together with a separator made in three layers of polypropylene/polyethylene/polypropylene (PP/PE/PP), and then the electrolyte prepared according to Examples 1-1 to 6-1 and Comparative Examples 1-1 to 6-3 was injected thereto to completely manufacture a lithium secondary battery.

### Experimental Example

In order to evaluate the performance of the secondary battery manufactured above, each performance was measured by the following method, and the results are shown in Tables 7 to 9 below.

### [Evaluation of HPPC discharge resistance]

The HPPC discharge resistance may be measured by the method specified in the document "Battery test manual for plug-in hybrid electric vehicles" (2010, Idaho National Laboratory for the U.S. Department of Energy).

After the secondary battery is left alone for 5 hours at 60°C, the measured voltage value, the charge and discharge current value corresponding to C-rate, the current change amount (△I), the discharge voltage change amount (△V), the charge voltage change amount (△V), the discharge resistance and the charge resistance were measured, and the resistance value was calculated using the slope value obtained from the current and voltage change amount by allowing the charge and discharge current for each C-rate to flow briefly for a certain period of time. The calculated resistance value is shown in Tables 7 and 8 below as the initial discharge resistance item.

### [Evaluation of high temperature recovery capacity]

The secondary battery was charged under the charge conditions of constant current of 1.0C and voltage of 4.2V until the charging current reached 1/10C. After charging and discharging were performed up to 3.0V under the discharge condition of constant current of 1.0C, the (initial) recovery capacity was measured.

The secondary battery was stored in a constant temperature bath at 60°C for 4 weeks after being charged under the same charging and discharging conditions, and then the secondary battery was discharged to a discharge voltage of 3V at a room temperature of 25°C, and then the remaining capacity was measured. After performing the above charging and discharging process 100 times under the same charging and discharging conditions, the recovery capacity was measured. The average value calculation result of the recovery capacity is shown in Tables 7 and 8 below as the item of the recovery capacity after high temperature storage.

### [Evaluation of negative electrode expansion]

The thickness of the secondary battery was measured using a compression type thickness meter, produced by Mitutoyo, with a pouch cell being placed between the compression plates and compressed with a weight of 300 g. In order to exclude the cooling effect, the thickness measured immediately after taking the secondary battery out of an oven at 60°C (expansion thickness) and the thickness measured in the same way after the secondary battery was stored in a constant temperature bath at 60°C for 4 weeks were put into Equation 1 below to obtain the thickness increase rate (%). The calculation results are shown in Tables 7 and 8 below.Thickness increase rate (%) = {(thicnkess after high temperature storage - initial thickness) / initial thickness) X 100

### [Evaluation of coulombic efficiency]

The secondary battery was charged at 45°C with a constant current of 1 C-rate until the voltage reached 4.20 V (vs. Li), and then cut-off at a current of 0.05 C-rate while maintaining 4.20 V in a constant voltage mode. Subsequently, the secondary battery was discharged performed at a constant current of 1 C-rate until the voltage reached 3.0V (vs. Li).

The coulombic efficiency was calculated by putting the charge capacity and discharge capacity after 300 cycles into Equation 2 below. The calculation results are shown in Tables 7 and 8 below.Coulombic efficiency (%) = (discharge capacity at 300 cycles / charge capacity at 300 cycles) x 100

### [Gas generation]

After 300 cycles mentioned above, the generation of CO₂ gas and H₂ gas due to the electrolyte decomposition and SEI film formation during the 300 cycle charge and discharge was confirmed by whether the cell thickness increased.

### [Evaluation of output characteristics]

The secondary battery was charged and discharged at room temperature for every 5 cycles at 1 C-rate, 2 C-rate, and 3 C-rate. The results obtained through the corresponding evaluation were put into Equation 3 below to calculate the rate efficiency of each composition. The obtained results are shown in Table 9 below. For example, when the discharge capacity for each C-rate is 1 C-rate discharge capacity, the charge capacity for each C-rate refers to 1 C-rate charge capacity. Rate efficiency(%) = (discharge capacity for each C-rate / charge capacity for each C-rate) X 100

**Table 7**

| | **First additive (wt%)** | **Second additive (wt%)** | **Initial resistance (mΩ)** | **Recovery capacity after high temperature storage (mAh)** | **Gas gener ation** | **Initial thickne ss (mm** | **Expande d thickness (mm)** | **Thickness increase rate (%)** | **Coulomb ic efficiency (%)** |
|---|---|---|---|---|---|---|---|---|---|
| Ex. 1-1 | Formula 1a (10) | Formula 2a+ cesium (5) | 25.59 | 604.3 | | 2.3 | 2.5 | 8.96 | - |
| Ex. 2-1 | Formula 1b (10) | Formula 2a+ cesium (5) | 27.35 | 591.7 | X | 2.3 | 2.5 | 8.96 | 99.8 |
| Ex. 2-2 | Formula 1c (10) | Formula 2a+ cesium (5) | 26.68 | 593.6 | X | 2.3 | 2.6 | 13.04 | 99.8 |
| Ex. 2-3 | Formula 1d (10) | Formula 2a+ cesium (5) | 26.24 | 586.6 | X | 2.3 | 2.4 | 4.35 | 99.8 |
| Ex. 3-1 | Formula 1e (10) | Formula 2a+ cesium (5) | 26.85 | 585.5 | | 2.3 | 2.4 | 4.35 | 99.7 |
| Ex. 4-1 | Formula 1f (10) | Formula 2a+ cesium (5) | 26.98 | 602.7 | X | 2.3 | 2.4 | 4.35 | 99.8 |
| Ex. 5-1 | Formula 1g (10) | Formula 2a+ cesium (5) | 27.65 | 594.1 | | 2.3 | 2.5 | 8.96 | 99.6 |
| Ex. 5-2 | Formula 1h (10) | Formula 2a+ cesium (5) | 26.89 | 593.6 | | 2.3 | 2.5 | 8.96 | 99.8 |
| Ex. 5-3 | Formula 1i (10) | Formula 2a+ cesium (5) | 26.13 | 583.7 | | 2.3 | 2.4 | 4.35 | 99.7 |
| Ex. 5-4 | Formula 1j (10) | Formula 2a+ cesium (5) | 26.32 | 585.5 | | 2.3 | 2.4 | 4.35 | 99.6 |
| Ex. 5-5 | Formula 1k (10) | Formula 2a+ cesium (5) | 26.95 | 579.1 | | 2.3 | 2.6 | 13.04 | 99.7 |
| Ex. 5-6 | Formula 1l (10) | Formula 2a+ cesium (5) | 26.65 | 588.6 | | 2.3 | 2.6 | 13.04 | 99.8 |
| Ex. 5-7 | Formula 1m (10) | Formula 2a+ cesium (5) | 26.32 | 591.7 | | 2.3 | 2.5 | 8.96 | 99.8 |
| Ex. 6-1 | Formula 1n (10) | Formula 2a+ cesium (5) | 27.11 | 606.5 | X | 2.3 | 2.5 | 8.96 | 99.8 |

**Table 8**

| | **First additive (wt%)** | **Second additive (wt%)** | **Initial resistance (mΩ)** | **Recovery capacity after high temperature storage (mAh)** | **Gas gener ation** | **Initial thickne ss (mm** | **Expande d thickness (mm)** | **Thickness increase rate (%)** | **Coulomb ic efficiency (%)** |
|---|---|---|---|---|---|---|---|---|---|
| Comp. Ex. 1-1 | Formula 1a (10) | - | 29.62 | 579.2 | 2.3 | | 2.8 | 21.74 | - |
| Comp. Ex. 1-2 | Formula 1a (10) | Formula 2a+ sodium (5) | 30.75 | 571.6 | 2.3 | | 2.7 | 17.39 | - |
| Comp. Ex. 1-3 | - | Formula 2a+ cesium (5) | 28.74 | 586.7 | 2.3 | | 2.8 | 21.74 | - |
| Comp. Ex. 2-2 | Formula 1b (5) | Formula 2a+ sodium (5) | 31.28 | 569.2 | 2.3 | X | 2.6 | 13.04 | 99.4 |
| Comp. Ex. 2-6 | Formula 1c (10) | Formula 2a+ sodium (5) | 28.24 | 568.7 | 2.3 | | 2.6 | 13.04 | 99.6 |
| Comp. Ex. 2-7 | Formula 1c (10) | - | 28.65 | 560.1 | 2.3 | | 2.7 | 17.39 | 99.5 |
| Comp. Ex. 2-9 | Formula 1d (10) | Formula 2a+ sodium (5) | 29.16 | 561.2 | 2.3 | | 2.7 | 17.39 | 99.4 |
| Comp. Ex. 2-10 | Formula 1d (10) | - | 30.15 | 559.1 | 2.3 | | 2.8 | 21.74 | 99.3 |
| Comp. Ex. 3-1 | Formula 1e (10) | - | 28.96 | 564.6 | 2.3 | | 2.8 | 21.74 | 99.4 |
| Comp. Ex. 3-2 | Formula 1e (10) | Formula 2a+ sodium (5) | 28.95 | 572.2 | 2.3 | | 2.7 | 17.39 | 99.6 |
| Comp. Ex. 4-2 | Formula 1f (10) | Formula 2a+ sodium (5) | 31.65 | 581.1 | 2.3 | | 2.7 | 17.39 | 99.2 |
| Comp. Ex. 4-3 | Formula 1f (10) | - | 29.56 | 586.8 | 2.3 | | 2.5 | 8.96 | 99.3 |
| Comp. Ex. 5-1 | Formula 1g (5) | Formula 2a+ sodium (5) | 29.65 | 575.6 | 2.3 | | 2.8 | 21.74 | 99.4 |
| Comp. Ex. 5-2 | Formula 1g (10) | - | 30.12 | 586.2 | 2.3 | | 2.7 | 17.39 | 99.5 |
| Comp. Ex. 5-3 | Formula 1h (10) | - | 30.65 | 571.9 | 2.3 | | 2.8 | 21.74 | 99.6 |
| Comp. Ex. 5-4 | Formula 1i (10) | - | 32.45 | 588.8 | 2.3 | | 2.7 | 17.39 | 99.5 |
| Comp. Ex. 5-5 | Formula 1j (10) | - | 29.98 | 566.7 | 2.3 | | 2.6 | 13.04 | 99.5 |
| Comp. Ex. 5-6 | Formula 1k (10) | Formula 2a+ sodium (10) | 30.25 | 559.3 | 2.3 | | 2.7 | 17.39 | 99.2 |
| Comp. Ex. 5-7 | Formula 1k (10) | - | 30.13 | 562.2 | 2.3 | | 2.8 | 21.74 | 99.3 |
| Comp. Ex. 5-8 | Formula 1l (10) | Formula 2a+ sodium (5) | 28.28 | 566.3 | 2.3 | | 2.9 | 26.09 | 99.6 |
| Comp. Ex. 5-9 | Formula 1l (10) | - | 29.31 | 568.7 | 2.3 | | 3.1 | 34.78 | 99.6 |
| Comp. Ex. 5-10 | Formula 1m (10) | - | 30.12 | 563.6 | 2.3 | | 2.9 | 26.09 | 99.7 |
| Comp. Ex. 6-2 | Formula 1n (10) | Formula 2a+ sodium (5) | 28.65 | 589.3 | 2.3 | | 2.8 | 21.74 | 99.6 |
| Comp. Ex. 6-3 | Formula 1n (10) | - | 31.25 | 591.6 | 2.3 | | 2.9 | 26.09 | 99.8 |

**Table 9**

| | First additive (wt%) | Second additive (wt%) | Rate efficiency 1C-rate (%) | Rate efficiency 2C-rate (%) | Rate efficiency 3C-rate (%) |
|---|---|---|---|---|---|
| Example 1-1 | Formula 1a (10) | Formula 2a+cesium (5) | 97.5 | 94.7 | 74.1 |
| Example 2-1 | Formula 1b (10) | Formula 2a+cesium (5) | 97.2 | 94.3 | 74.4 |
| Example 2-2 | Formula 1c (10) | Formula 2a+cesium (5) | 97.5 | 94.5 | 76.0 |
| Example 2-3 | Formula 1d (10) | Formula 2a+cesium (5) | 97.2 | 95.1 | 74.2 |
| Example 3-1 | Formula 1e (10) | Formula 2a+cesium (5) | 97.4 | 94.7 | 74.9 |
| Example 4-1 | Formula 1f (10) | Formula 2a+cesium (5) | 97.4 | 94.6 | 74.4 |
| Example 5-1 | Formula 1g (10) | Formula 2a+cesium (5) | 97.5 | 94.5 | 74.2 |
| Example 5-2 | Formula 1h (10) | Formula 2a+cesium (5) | 97.6 | 94.9 | 77.1 |
| Example 5-3 | Formula 1i (10) | Formula 2a+cesium (5) | 97.4 | 95.3 | 74.4 |
| Example 5-4 | Formula 1j (10) | Formula 2a+cesium (5) | 97.4 | 94.7 | 74.6 |
| Example 5-5 | Formula 1k (10) | Formula 2a+cesium (5) | 97.4 | 94.9 | 76.6 |
| Example 5-6 | Formula 1l (10) | Formula 2a+cesium (5) | 97.6 | 94.5 | 74.8 |
| Example 5-7 | Formula 1m (10) | Formula 2a+cesium (5) | 97.7 | 94.8 | 74.5 |
| Example 6-1 | Formula 1n (10) | Formula 2a+cesium (5) | 97.5 | 94.6 | 74.6 |
| Comparative Example 1-1 | Formula 1a (10) | - | 97.2 | 94.6 | 72.5 |

As shown in Tables 7 and 8, in the secondary batteries of Examples 1-1 to 6-1 using the electrolyte additive of the present disclosure, the charge resistance value is found to be 25.59 to 27.65 mQ, but in Comparative Examples 1-1 to 6-3 using only one of the first additive and the second additive, the charge resistance is found to be as high as 28.24 to 31.65 mQ, so it may be understood that the charge resistance value is lowered by up to 23.6% by using the electrolyte additive of the present disclosure. In addition, as shown in Table 9, in the secondary batteries of Examples 1-1 to 6-1 using the electrolyte additive of the present disclosure, it is found that the rate efficiency 3 C-rate (high rate charge and discharge characteristics) are increased by 2 to 4% compared to Comparative Example 1-1. This indicates that the electrolyte additive of the present disclosure has the effect of improving battery output. In addition, as shown in Tables 7 and 8, in the secondary batteries of Examples 1-1 to 6-1 using the electrolyte additive of the present disclosure, the high temperature recovery capacity is 585.5 to 606.5 mAh, whereas in Comparative Examples 1-1 to 6-3 using only one of the first additive and the second additive, the high temperature recovery capacity is 559.1 to 591.6 mAh, which is lower than that of the examples of the present disclosure by up to 47.4 mAh. This means that the recovery capacity at high temperature of 60°C is improved by the electrolyte additive of the present disclosure, and it may be found that the recovery capacity efficiency of the battery is improved when the secondary battery is stored for a long time in a high temperature environment by the electrolyte additive of the present disclosure.

In addition, as a result of checking the negative electrode expansion, it may be found that in the secondary batteries of Examples 1-1 to 6-1 using the electrolyte additive of the present disclosure, the thickness increase rate is 2.4 to 2.6%, whereas in Comparative Examples 1-1 to 6-3 using only one of the first additive and the second additive, the thickness increase rate is 2.6 to 3.1%, which is lower than that of the examples of the present disclosure by up to 0.7 %p (% points). This means that by using the electrolyte additive of the present disclosure, the capacity retention of the battery is improved while repeating the charging and discharging process for 300 cycles at a high temperature of 60°C compared to the case of using the conventional electrolyte additive, and thus it is found that the temperature storage and the lifespan performance are improved by using the electrolyte additive of the present disclosure.

In addition, as a result of the coulombic efficiency evaluation, in the secondary batteries of Examples 1-1 to 6-1 using the electrolyte additive of the present disclosure, the coulombic efficiency is 99.6 to 99.8%, whereas in Comparative Examples 1-1 to 6-3, the coulombic efficiency is 99.2 and 99.8%, which is lower than that of the examples of the present disclosure by up to 0.3 %p (% points). This means that by using the electrolyte additive of the present disclosure, the capacity retention of the battery is improved while repeating the charging and discharging process for 300 cycles at a high temperature of 60°C compared to the case of using the conventional electrolyte additive, and thus it is found that the temperature storage and the lifespan performance are improved in a high temperature environment by using the electrolyte additive of the present disclosure.

Therefore, if the electrolyte additive of the present disclosure and the electrolyte containing the electrolyte additive are applied to a secondary battery, the discharge resistance, output, recovery capacity and lifespan efficiency are improved, and thus it may be understood that the secondary battery is suitable for use as a secondary battery for energy storage systems (ESS), vehicles, or the like.

## Claims

1. An electrolyte, which includes an organic solvent, lithium salt, a first additive and a second additive,
wherein the first additive contains a compound having an atomic group with 3 to 5 atoms represented by Formula 1 or 2 in an amount of 15 weight% or less based on 100 weight% of the electrolyte, the atomic group having 2 to 4 atoms with electronegativity of 3 or more and having at least one double bond,
wherein the second additive contains a compound having an atomic group with 3 to 5 atoms and a symmetric structure in an amount of 0.01 to 10 weight% based on 100 weight% of the electrolyte, the atomic group having 2 to 4 atoms with electronegativity of 3 or more and having at least one double bond: 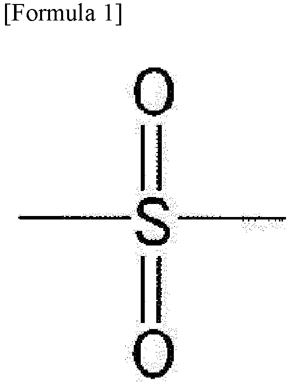 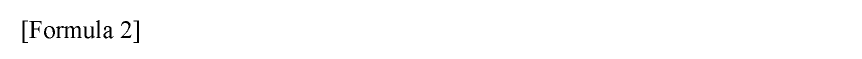 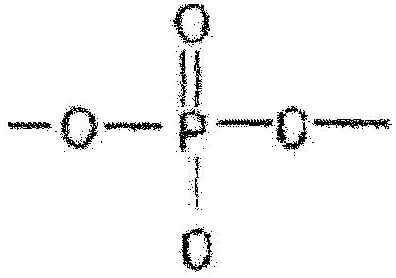
wherein in Formulas 1 and 2, a solid line is a bond.

2. The electrolyte according to claim 1,
wherein the first additive is at least one selected from compounds represented by Formulas 3 to 6 below: 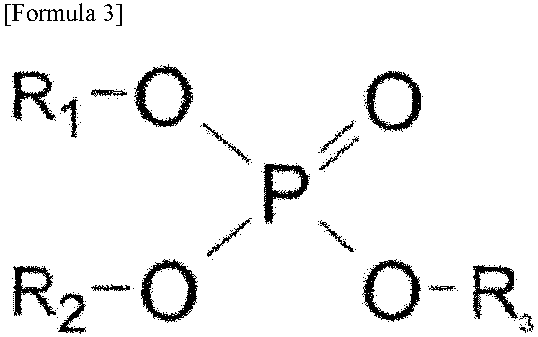
wherein in Formula 3, R₁, R₂ and R₃ are independently an alkyl group with 1 to 5 carbon atoms or an aryl group with 6 to 10 carbon atoms, 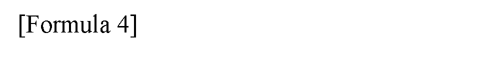 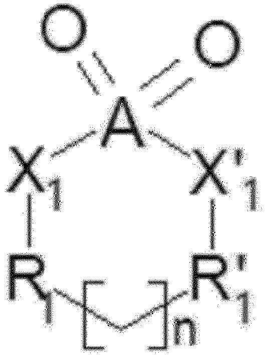 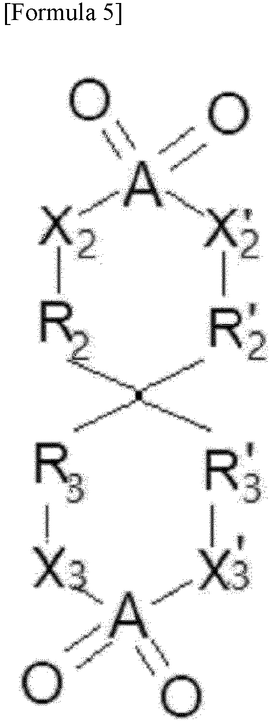
wherein in Formulas 4 to 5, A is phosphorus (P), sulfur (S), or nitrogen (N); X₁, X₂, X₃, X₁', X₂' and X₃' are independently a bond or oxygen; n is an integer of 0 to 3; and Ri, R₂, R₃, R₁', R₂' and R₃' are independently a bond, substituted or unsubstituted alkylene with 1 to 10 carbon atoms and have at least one carbon, 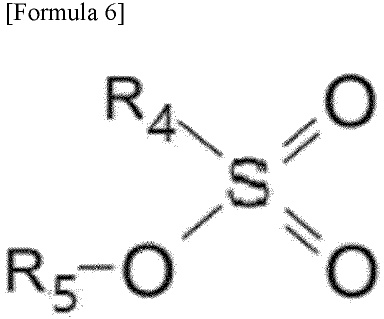
wherein in Formula 6, R₄ is OR₆ or R₆; R₅ is R₇A; A is 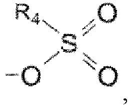 and R₆ and R₇ are independently an alkyl group with 1 to 5 carbon atoms or an aryl group with 6 to 10 carbon atoms.

3. The electrolyte according to claim 1,
wherein the first additive is at least one selected from compounds represented by Formulas 7 to 13 below: 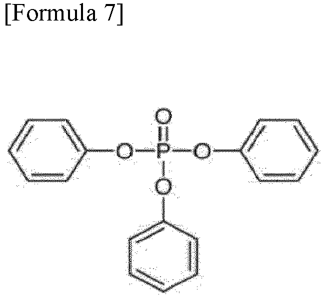 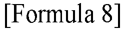 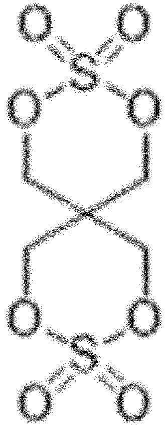 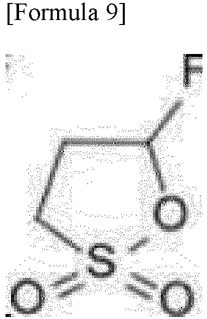 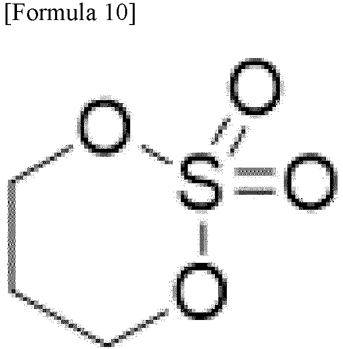 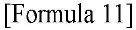 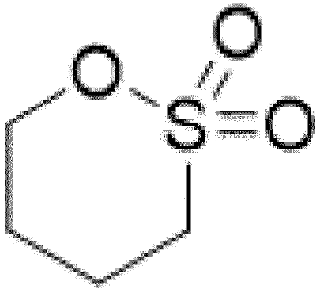 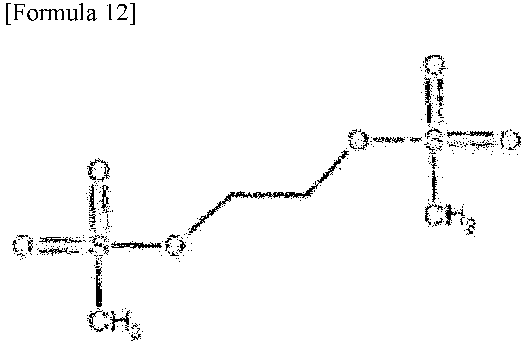 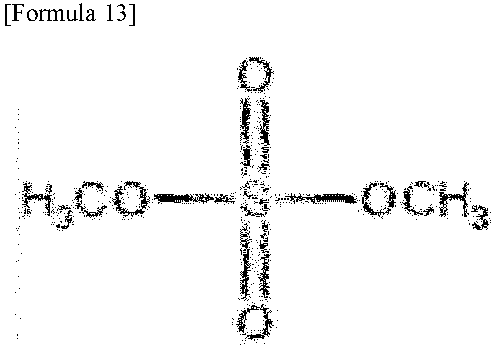
wherein in Formulas 7 to 13, a solid line is a bond; a point where a bond meets a bond is carbon when a separate element is not described; and the number of hydrogens satisfying the atom value of the carbon is omitted.

4. The electrolyte according to claim 1,
wherein the second additive is a compound containing a pair of at least one cation selected from the group consisting of alkali metal ions (excluding lithium) and an anion represented by Formula 14 below: 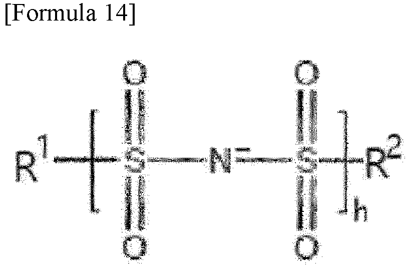
wherein in Formula 14, R¹ and R² are independently hydrogen, halogen, or a straight-chain or branched alkyl group containing a halogen substituent with 1 to 7 carbon atoms, and h is an integer of 1 to 10.

5. The electrolyte according to claim 4,
wherein in the anion represented by Formula 14, the halogen substituent is fluorine.

6. The electrolyte according to claim 1,
wherein the second additive is a compound containing a pair of a cation of cesium or sodium and at least one anion selected from the group represented by Formulas 15 to 17 below: 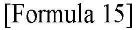 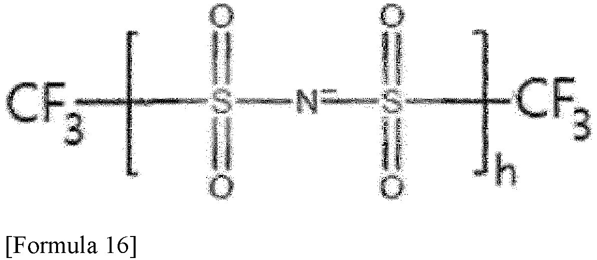 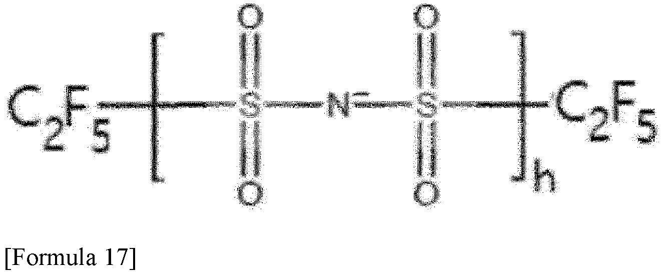 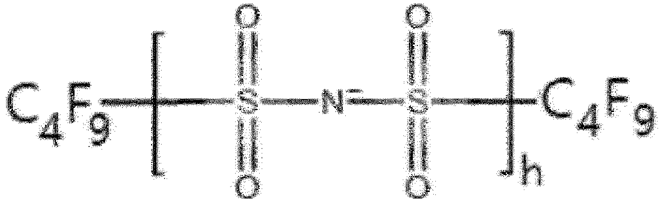
wherein in Formulas 15 to 17, h is an integer of 1 to 10.

7. The electrolyte according to claim 1,
wherein the first additive and the second additive are included at a weight ratio of 1:0.5 to 2.5 (first additive: second additive).

8. The electrolyte according to claim 1,
wherein the lithium salt includes at least one selected from the group consisting of LiPF₆, LiClO₄, LiAsF₆, LiBF₄, LiBF₆, LiSbF₆, LiA0₄, LiAlCl₄, LiClO₄, LiCF₃SO₃, LiC₄F₉SO₃, LiN(C₂F₅SO₃)₂, LiN(C₂F₅SO₂)₂, LiN(CF₃SO₂)₂,LiN(CaF₂ₐ₊₁SO₂) (C_{b}F_{2b+1}SO₂) (where a and b are natural numbers), LiCl, LiI, and LiB(C₂O₄)₂.

9. The electrolyte according to claim 1,
wherein the organic solvent includes at least two selected from the group consisting of ethylene carbonate (EC), diethyl carbonate (DEC), ethylmethyl carbonate (EMC), dimethyl carbonate (DMC), propylene carbonate (PC), dipropyl carbonate (DPC), butylene carbonate, methylpropyl carbonate, ethylpropyl carbonate, methyl propionate (MP), ethyl propionate (EP), and propyl propionate (PP).

10. The electrolyte according to claim 1, further comprising:
at least one third additive selected from the group consisting of boron compound, phosphorus compound, sulfur compound, and nitrogen compound in an amount of 10 weight% or less based on a total of 100 weight% of the electrolyte.

11. An electrolyte additive, comprising:
at least one compound selected from compounds represented by Formulas 3 to 6 below; and
a compound containing a pair of cesium cation and an anion represented by Formula 14 below: 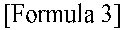 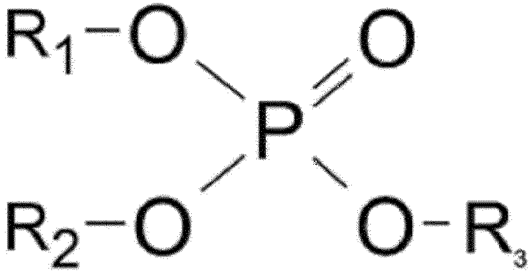
wherein in Formula 3, Ri, R₂ and R₃ are independently an alkyl group with 1 to 5 carbon atoms or an aryl group with 6 to 10 carbon atoms, 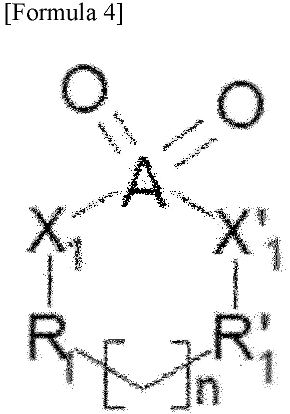 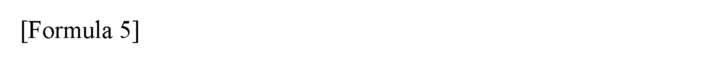 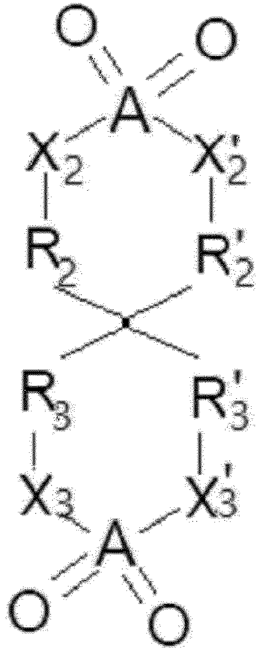
wherein in Formulas 4 to 5, A is phosphorus (P), sulfur (S), or nitrogen (N); X₁, X₂, X₃, X₁', X₂' and X₃' are independently a bond or oxygen; n is an integer of 0 to 3; and Ri, R₂, R₃, R₁', R₂' and R₃' are independently a bond, substituted or unsubstituted alkylene with 1 to 10 carbon atoms and have at least one carbon, 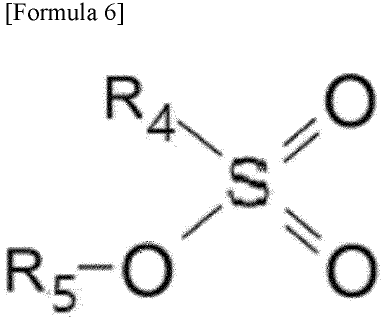
wherein in Formula 6, R₄ is OR₆ or R₆; R₅ is R₇A; A is 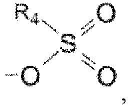 and R₆ and R₇ are independently an alkyl group with 1 to 5 carbon atoms or an aryl group with 6 to 10 carbon atoms, 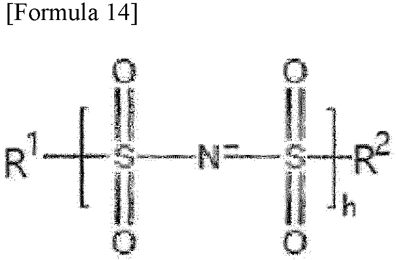
wherein in Formula 14, R¹ and R² are independently hydrogen, halogen, or a straight-chain or branched alkyl group containing a halogen substituent with 1 to 7 carbon atoms, and h is an integer of 1 to 10.

12. The electrolyte additive according to claim 11,
wherein the at least one compound selected from compounds represented by Formulas 3 to 6 and the compound containing a pair of cesium cation and an anion represented by Formula 14 are included at a weight ratio of 1:0.5 to 2.5 (first additive: second additive).

13. A lithium secondary battery, which includes a negative electrode, a positive electrode, a separator interposed between the negative electrode and the positive electrode, and an electrolyte,
wherein the electrolyte is an electrolyte according to any one of claims 1 to 10.

14. The lithium secondary battery according to claim 13,
wherein the secondary battery has a discharge resistance value of 28 mΩ or less at 60°C.

15. The lithium secondary battery according to claim 13,
wherein the secondary battery has a recovery capacity of 585 mAh or more at 60°C.

16. The lithium secondary battery according to claim 13,
wherein the secondary battery has a thickness increase rate of 2.6% or less at 60°C calculated using Equation 1 below: Thickness increase rate (%) = {(thickNess after high temperature storage - initial thicKness) / initial thickness) X 100.

17. The lithium secondary battery according to claim 13,
wherein the secondary battery has a coulombic efficiency of 99% or more calculated using Equation 2 below after 300 cycles: Coulombic efficiency (%) = (discharge capacity at 300 cycles / charge capacity at 300 cycles) x 100.

18. The lithium secondary battery according to claim 13,
wherein the secondary battery is a battery for an energy storage system (ESS) or a vehicle.
